Europäisches Patentamt

European Patent Office

Office européen des brevets

(19)

(11) Numéro de publication: **0 033 686**
**B1**

(12) **FASCICULE DE BREVET EUROPEEN**

(45) Date de publication du fascicule du brevet: **21.11.84**

(21) Numéro de dépôt: **81400116.0**

(22) Date de dépôt: **27.01.81**

(51) Int. Cl.[3]: **A 23 J 3/02,** A 23 C 9/12,
A 23 C 9/142, A 61 K 37/18

(54) Procédé de traitement d'une matière première à base de caséine, contenant des phosphocaséinates de cations bivalents, produits obtenus et applications.

(30) Priorité: **01.02.80 FR 8002280**

(43) Date de publication de la demande:
**12.08.81 Bulletin 81/32**

(45) Mention de la délivrance du brevet:
**21.11.84 Bulletin 84/47**

(84) Etats contractants désignés:
**AT BE CH DE FR GB IT LI LU NL SE**

(56) Documents cités:
FR-A-2 292 435
FR-A-2 399 213

CHEMICAL ABSTRACTS, vol. 87, no. 19, 7
novembre 1977, page 265, abrégé 148285p
Columbus, Ohio, US D.W. WEST: "A simple
method for the isolation of a phosphopeptide
from bovine alpha s1-casein"
CHEMICAL ABSTRACTS, vol. 91, no. 21, 19
novembre 1979, page 523 abrégé 173597g
Columbus, Ohio, US J.P. ROOZEN et al.:
"Enzymic protein hydrolysis in a membrane
reactor related to taste properties"

(73) Titulaire: **INSTITUT NATIONAL DE LA
RECHERCHE AGRONOMIQUE (INRA)
149, rue de Grenelle
F-75341 Paris Cedex 07 (FR)**

(72) Inventeur: **Brule, Gérard
5, Cours de Lisbonne
F-35100 Rennes (FR)**
Inventeur: **Roger, Loic
27, rue de Brest
F-35000 Rennes (FR)**
Inventeur: **Fauquant, Jacques
Le Ricandais
Pleumeleuc F-35160 Montfort (FR)**
Inventeur: **Piot, Michel
10, rue du Bourbonnais
F-35000 Rennes (FR)**

(74) Mandataire: **Phélip, Bruno et al
c/o Cabinet Harlé & Phélip 21, rue de la
Rochefoucauld
F-75009 Paris (FR)**

EP 0 033 686 B1

Courier Press, Leamington Spa, England.

**0 033 686**

## Description

L'invention concerne le domaine du traitement de matières à base de caséine. Elle a plus particulièrement pour objet un procédé de traitement d'une telle matière contenant des phosphocaséinates de cations bivalents, tels que les phosphocaséinates de calcium et/ou de magnésium. De telles substances se trouvent présentes, par exemple, dans le lait ou dans des matières premières laitières, telles que les rétentats d'ultrafiltration du lait. L'invention concerne également les produits obtenus par un tel procédé, en particulier des phosphopeptides et des peptides non phosphorylés. L'invention a encore pour objet les applications des produits ainsi obtenus, spécialement celles des phosphopeptides qui peuvent être avantageusement utilisés dans le domaine alimentaire pour répondre à des besoins spécifiques nutritionnels.

On sait que les caséines des matières premières laitières, et tout particulièrement du lait, contiennent des phosphosérines qui confèrent aux peptides dans lesquels elles se trouvent des propriétés physico-chimiques, technologiques et physiologiques intéressantes. On trouvera notamment des indications sur les protéines du lait, dans l'ouvrage de McKenzie H. A. (1971) intitulé "Milk proteins" Vol. 1 et 2 Academic Press New-York.

L'ultrafiltration sur membranes, étant donné les progrès réalisés tant dans les appareillages que dans la compréhension des phénomènes observés, s'est largement répandue dans l'industrie laitière, pour le traitement du lait (voir par exemple Maubois J. L., Mocquot G. (1971)—Préparation de fromages à partir de pré-fromages liquides obtenus par ultrafiltration du lait—Revue "Le Lait" fascicule *51* 508, 495—533). Lors du passage du lait sur la membrane d'ultrafiltration, l'eau, les sels minéraux solubles, le lactose, les composés azotés de faible poids moléculaire (peptides, acides aminés libres) et les vitamines hydrosolubles, traversent la membrane sous forme d'ultrafiltrat ou perméat, alors que les protéines et les constituants associés (calcium, phosphore), les globules gras et les éléments lipophiles sont retenus et se concentrent au fur et à mesure de l'élimination de la phase aqueuse; ils constituent le rétentat ou concentré protéique. L'obtention de concentrés protéiques de pureté élevée, nécessite la mise en oeuvre d'une ultrafiltration ainsi que d'une diafiltration. Dans la diafiltration, on effectue une addition d'eau ou de solution aqueuse contenant des sels, en continu ou en discontinu, dans le rétentat d'ultrafiltration. On élimine simultanément on successivement une quantité équivalente de perméat. Une telle opération a pour conséquence d'appauvrir le rétentat en éléments filtrables. L'avantage de la technique d'ultrafiltration sur membranes est de conserver les protéines du lait à leur état natif.

Le procédé de l'invention tire profit de l'ultrafiltration sur membranes pour opérer un fractionnement des constituants des matières premières à base de caséine, mais en combinant cette ultrafiltration à une opération d'hydrolyse enzymatique.

On connaît un certain nombre de procédés pour l'hydrolyse des protéines, par exemple des protéines de lait. L'hydrolyse acide conduit effectivement à l'obtention de solutions d'acides aminés libres, mais elle en détruit certains. L'hydrolyse alcaline préserve le tryptophane mais entraîne une insolubilisation qui diminue fortement la valeur nutritionnelle des concentrés protéiques initiaux.

La protéolyse enzymatique est connue et pratiquée depuis fort longtemps à des fins analytiques ou à des fins alimentaires, le but principal étant de solubiliser les protéines. La littérature fait largement état de nombreuses utilisations alimentaires d'hydrolysats de protéines de soja [Voir Arai. S., Noguchi M., Kurosawa S., Kato H. et Fujimaki M. (1970) Applying proteolytic enzymes on soybean, 6-Deodorization effect], des protéines de poissons: [voir Hevia P., Whitaker J. R. et Olcott H. S. (1976)—Solubilization of a fish protein concentrate with proteolytic enzymes. J. Agric. Food Chem. Vol. 24 (2) 383—385] ou de colza, par action de protéases animale, microbienne ou végétale.

L'application de ces techniques aus protéines laitières au niveau industriel reste cependant très limitée.

La protéolyse enzymatique ne présente pas les inconvénients des méthodes chimiques. Les conditions d'hydrolyse sont modérées et préservent anisi la qualité nutritionnelle des produits.

En général, l'hydrolyse conduit à des peptides ayant un goût amer prononcé. Ce caractère limite l'utilisation de ces hydrolysats en alimentation humaine. L'intensité de l'amertume d'un hydrolysat dépend principalement de la nature du substrat protéique et de la spécificité des enzymes. Pour éliminer l'amertume, on a suggéré de faire agir des exopeptidases. Voir par exemple Arai S., Yamashita M., Kato H., Fujimaki M. (1970)—Agric. Biol. Chem. 34, 729, ainsi que Clegg K. M., Smith G. et Walker A. L. (1974)—Production of an enzymatic hydrolysate of casein on a kilogram scale. J. Food Technol. *9*, 425—431. On a également proposé de modifier les peptides par addition d'acide glutamique avant réaction plastéique. Il est également possible de recourir à une élimination des acides aminés hydrophobes.

Cependant, toutes ces techniques connues ne sont pas satisfaisantes et ne peuvent pas répondre aux besoins de l'invention. En effet, une solubilisation poussée, causée par la mise en oeuvre d'exopeptidase, augmente le taux d'acides aminés libres et plus spécialement l'arginine, la lysine, la tyrosine, la valine, la phênylalanine, la méthionine et la leucine, ce qui aurait pour effet direct d'encombrer les systèmes de transport, au niveau de la barrière intestinale, des acides aminés libres, et donc d'entraîner une moindre efficacité nutritionnelle des hydrolysats. Par ailleurs, la qualité intrinsèque

2

**O 033 686**

des hydrolysats est modifiée car l'équilibre en acides aminés est lui-même changé, ce qui nécessite une supplémentation en acides aminés libres.

Sur le plan technologique, l'hydrolyse enzymatique fait le plus souvent appel à un système de réacteur discontinu. L'enzyme est ajoutée à la solution protéique à traiter. Après un temps de séjour plus ou moins long, dans des conditions favorables à l'activité enzymatique et à l'attaque du substrat, le pH est modifié et un traitement thermique léger inactive l'enzyme. Une centrifugation peut être faite pour éliminer la fraction insoluble non digérée. Mais, selon cette technique de réaction d'hydrolyse enzymatique discontinue, il est difficile d'utiliser un rapport enzyme/substrat élevé. Or, on connaît, voir Robins R. C. (1978)—Effect of ratio of enzymes to substrate on amino acid patterns released from proteins in vitro. Internat. J. Vit. Nutr. Res. *48*, 44—52—, l'influence décisive du rapport enzyme/substrat sur la nature des acides aminés libres et les peptides libérés lors de la protéolyse. Avec un procédé discontinu, il faut détruire les enzymes en fin d'hydrolyse lorsque celles-ci sont en excès, ce qui serait indispensable avec les rapports élevés précités.

On a également proposé d'utiliser des réacteurs à enzymes fixées. Cependant, ceux-ci présentent de notables inconvénients sur le plan pratique. En effet, les conditions optimales d'activité des enzymes, en particulier les conditions de pH, se déplacent, de sorte que le fonctionnement du réacteur ne donne pas satisfaction en permanence. On constate également des problèmes bactériologiques, le colmatage des lits de fixation ainsi que l'adsorption des protéines sur le support. En outre, la réaction enzymatique a tendance à s'inhiber au cours du temps en raison de la formation de complexe enzyme-fragment protéique. L'inhibition peut être également due à la nature du support. Il est par ailleurs très difficile d'utiliser des systèmes multi-enzymes en raison des phénomènes de compétition des enzymes vis-à-vis du support et de la stabilité différente des enzymes au cours du temps.

L'invention a mis a profit les moyens déjà connus dans certaines autres applications et qui consistent à faire appel à des réacteurs enzymatiques à membranes. On peut se référer par exemple à l'article de Cheftel C. (1972)—Solubilisation enzymatique continue du concentré protéique de poisson. Essai de recyclage des enzymes. Ann. Technol. Agric. *21*, (3) 423—433-qui décrit un réacteur à membranes, appliqué à la protéolyse de concentrés protéiques de poisson. La membrane d'ultrafiltration permet de retenir l'enzyme en solution dans le réacteur ainsi que le substrat protéique. Seuls les produits de l'hydrolyse, les peptides, sont éliminés au fur et à mesure de leur formation. Cependant, dans la pratique, la mise en oeuvre d'un tel réacteur n'est pas aisée, comme le souligne Cheftel. Le substrat doit être complètement solubilisable par l'enzyme et la solution protéique doit présenter une qualité bactériologique irréprochable.

A titre de documents illustrant l'état de la technique, on peut encore citer les références ci-après:

le brevet Français 74 39311 (publication FR—A—2.292.435) concerne l'obtention de phosphocaséinate de calcium à partir d'un rétentat d'ultrafiltration du lait. L'enseignement de ce brevet a donc pour objet la production de phosphocaséinate de calcium, sans indiquer les moyens de fractionnement de celui-ci;

le brevet Français 77 24069 (publication FR—A—2.399.213) décrit le traitement d'un hydrolysat par ultrafiltration puis par électrodialyse. Ce document matérialise le fait qu'il était connu d'ultrafiltrer un hydrolysat protéique. Le procédé décrit dans ce brevet permet la production d'une solution pure d'acides aminés naturels;

la référence Chemical Abstracts, volume 87, No. 19, 7 Novembre 1977, page 265, abrégé 148 285p Columbus Ohio (US) & J. Dairy Research, volume 44, No. 2 (1977) pages 373—376 D. W. West "A simple method for the isolation of a phosphopeptide from bovine $\alpha$ sl-casein", décrit l'obtention d'un phosphopeptide à partir de caséinate. Le procédé implique une hydrolyse enzymatique par la trypsine, et des fractionnements par filtration sur gel et chromatographie, mais il débute par une réaction avec CNBr conduisant à des produits tout à fait spécifiques;

la référence Chemical Abstracts, volume 91, No. 21, 19 Novembre 1979, page 523, abrégé 173 597g Columbus Ohio (US) & Enzyme Microb. Technol. vol. 1, No. 2 (1979) pages 122—124 J. P. Roozen et al. "Enzymic protein hydrolysis in a membrane reactor related to taste properties" décrit l'hydrolyse en réacteur enzymatique, dans le but d'améliorer le goût des hydrolysats protéiques. Ce document matérialise donc le fait que le réacteur enzymatique est un appareil connu.

La présente invention a pour objet un procédé de traitement d'une matière première à base de caséine, dans laquelle les ions bivalents, tels que le calcium et/ou le magnésium, sont complexés par les phosphosérines, comme c'est le cas par exemple du lait et du rétentat d'ultrafiltration du lait. Le procédé de l'invention est basé sur le fait que les phosphosérines présentent une aptitude à complexer les ions alcalino-terreux, tels que les ions calcium et/ou magnésium, de sorte que l'on peut séparer, dans les produits résultant de l'hydrolyse enzymatique, les phosphopeptides et les peptides non phosphorylés. La complexation des cations bivalents facilite l'agglomération des phosphopeptides entre eux, ce qui permet de les séparer des peptides non phosphorylés.

Sous sa forme la plus générale, l'invention a pour objet un procédé pour le traitement d'une matière première à base de caséine contenant des phosphocaséinates de cations bivalents, tels que les phosphocaséinates de calcium et/ou magnésium, caractérisé en ce qu'on soumet la matière première à une hydrolyse enzymatique à l'aide d'au moins une enzyme protéolytique capable de reconstituer la digestion protéique qui prend place in vivo dans l'organisme humain, en ce qu'on récupère l'hydrolysat

3

**0 033 686**

obtenu, en ce qu'on soumet celui-ci à au moins une ultrafiltration sur des membranes capables de retenir les phosphopeptides, tout en laissant passer les peptides, l'ultrafiltrat contenant ainsi les peptides non phosphorylés, en ce qu'on récupère le rétentat d'ultrafiltration, en ce qu'on effectue un traitement de désagrégation des phosphopeptides contenus dans le rétentat, et en ce qu'on soumet celui-ci à au moins une nouvelle ultrafiltration sur des membranes ne retenant pas les phosphopeptides, lesquels sont ainsi séparés de l'enzyme et peuvent être récupérés en tant que produit.

Les matières premières susceptibles d'être soumises au procédé de l'invention sont des matières à base de caséine d'origine quelconque, contenant des phosphocaséinates de cations bivalents. Le lait naturel constitue une source convenable dans laquelle les phosphocaséinates de calcium et de magnésium se trouvent présents tous les deux. Une forme plus purifiée de matière première convenant aux besoins de l'invention est constituée par un rétentat d'ultrafiltration du lait, lequel contient aussi les phosphocaséinates d'alcalino-terreux. Il est également possible d'utiliser comme matière première des phosphocaséinates purifés au préalable par tout moyen connu de l'homme de l'art, du type des phosphocaséinates d'alcalino-terreux, par exemple de magnésium ou de calcium, qui sont aptes à être traités selon l'invention.

Si l'on utilise un rétentat d'ultrafiltration du lait comme matière première dans le procédé, l'ultrafiltration préalable qui permet de produire le rétentat n'a pas besoin d'être poussée à un taux de concentration élevé. Par exemple, des taux de concentration de l'ordre de 1,5 à 3 sont convenables. Dans la pratique, on choisit un taux de concentration de l'ordre de 2. On peut également faire suivre l'ultrafiltration proprement dite d'une diafiltration qui permet de purifier le rétentat et d'augmenter le rapport protéines/matières sèches.

Quelle que soit la matière première utilisée, elle est soumise, conformément au procédé de l'invention, à une hydrolyse enzymatique à l'aide d'au moins une enzyme protéolytique. Ainsi qu'on l'a mentionné précédemment, une telle hydrolyse est avantageusement mise en oeuvre dans un dispositif qui combine un équipement d'ultrafiltration à un réacteur enzymatique permettant ainsi une réalisation continue.

Dans un tel mode de réalisation, l'hydrolyse enzymatique est réalisée en continu en introduisant la matière première à base de caséin dans une zone de réaction en vue d'une mise en contact intime avec l'enzyme, on soutire en continu le produit de réaction en l'amenant de la zone de réaction dans une zone d'ultrafiltration, et l'on soutire de celle-ci, également en continu, un perméat qui constitue l'hydrolysat peptidique.

Dans le cadre du procédé de l'invention, l'hydrolyse enzymatique est réalisée sur des solutions protéiques dans lesquelles les ions bivalents, en particulier le calcium et/ou le magnésium, sont complexés par les phosphosérines. Au cours de l'hydrolyse enzymatique, le pH doit être ajusté entre 7 et 9. A cet effet, on introduit en continu ou en discontinu dans la zone de réaction, un composé basique qui peut être l'hydroxyde ou le carbonate de sodium, l'hydroxyde ou le carbonate de potassium, l'hydroxyde de calcium, l'hydroxyde d'ammonium ou un mélange de ces produits. Le choix d'un composé basique particulier dépendra de la destination du produit final.

A titre d'enzyme, on met en oeuvre de préférence au moins une enzyme protéolytique capable de reconstituer la digestion protéique qui prend place in vivo dans l'organisme humain. On utilise donc avantageusement la pancréatine, qui est un mélange complexe contenant de la trypsine, de la chymotrypsine et d'autres enzymes protéolytiques secondaires. Dans la pratique, on peut faire appel à un extrait pancréatique naturel disponible sur le marché et aisément accessible. Toutefois, si besoin est, on peut également mettre en oeuvre des enzymes résultant d'un mélange synthétique, par exemple d'alphachymotrypsine et de trypsine. De préférence, on utilise un mélange synthétique dont la composition se rapproche de la pancréatine, avec par conséquent présence des enzymes secondaires contenues dans l'extrait pancréatique naturel. On a trouvé selon l'invention qu'au pH compris entre 7 et 9, et de préférence entre 7 et 8,5, par exemple à 8, la pancréatine et autres enzymes analogues répondant aux besoins de l'invention présentaient une stabilité maximale.

Il convient d'observer également des conditions assez strictes de température dans la zone d'hydrolyse enzymatique. On a constaté en effet que l'activité des enzymes était davantage influencée par la température que par le pH. Des essais ont montré notamment, selon l'invention, qu'avec la trypsine, la température maximale au cours de l'hydrolyse enzymatique ne devait pas être supérieure à 54°C, et avec la chymotrypsine cette température ne devait pas dépasser 45°C. Dans la pratique, lorsque l'on utilisera la pancréatine, on adoptera un compromis tenant compte à la fois des conditions optimales de la protéolyse intestinale in vivo (température de l'ordre de 37°C) et de faut que des températures plus élevées sont moins favorables au développement des germes et permettent d'obtenir des débits d'ultrafiltration supérieurs. En général, on choisit des températures de l'ordre de 37 à 40°C, et de préférence encore voisines de 37°C.

Il va sans dire que les paramètres de la réaction, c'est-à-dire le pH et la température d'hydrolyse enzymatique, sont liés. L'homme de l'art pourra donc choisir les conditions les plus favorables dans chaque cas d'espèce.

Pour réaliser une hydrolyse enzymatique optimale, il convient également de choisir avec soin la membrane d'ultrafiltration à utiliser en relation avec le réacteur enzymatique. Les membranes utilisées

4

sont de type quelconque, organique ou inorganique. Une organisation des membranes ayant donné de bons résultats est celle des modules à fibres creuses. A titre particulier, on peut utiliser les membranes de la Société Amicon disponibles sur le marché sous la dénomination H10P5 (seuil de coupure 5000) et H10P10 (seuil de coupure 10.000) ainsi que les membranes de la Société Romicon disponibles sur le marché sous la dénomination PM2 (seuil de coupure 2000) ou PM50 (seuil de coupure 50.000). La seule condition à respecter est que la membrane, en fonctionnement, retienne efficacement l'enzyme, tout en présentant des performances satisfaisantes, en particulier sur le plan de la longévité.

Le procédé de l'invention peut être mis en oeuvre en deux étapes séparées: une première étape consistant en l'hydrolyse enzymatique, et une deuxième étape consistant en l'ultrafiltration associée à ladite hydrolyse. Les équipements pour la mise en oeuvre de chacune de ces opérations peuvent être séparés ou intégrés. Mais, en variante, le procédé peut également être exécuté en continu, les deux étapes ci-dessus étant réalisées dans un appareillage unique. Pendant les premiers instants de fonctionnement, par exemple pendant une heure environ, le perméat (liquide passant à travers la membrane) est recyclé dans la zone d'hydrolyse afin d'obtenir le taux d'hydrolyse désiré de la matière à base de caséine. Après l'hydrolyse, on introduit dans le réacteur la matière première à base de caséine à traiter à un débit identique à celui du perméat.

Dans la pratique, on peut utiliser du lait cru ou écrémé, de n'importe quelle origine. L'hydrolyse est avantageusement effectuée en réacteur enzymatique à 37°C en présence de 2 g par litre d'enzyme pancréatique sur du lait cru écrémé dont le pH est ajusté à 8 par addition d'hydroxyde de sodium.

En variante, on peut traiter un lait cru et écrémé, concentré au préalable par ultrafiltration jusqu'à un taux de concentration voisin de 2. On porte alors le pH de ce rétentat à une valeur voisine de 8 par addition d'hydroxyde de sodium et on le soumet à une hydrolyse en réacteur enzymatique à 37°C en présence de 4 g/litre d'enzyme pancréatique.

Après l'hydrolyse, la solution résiduelle du réacteur enzymatique est concentrée par ultrafiltration. Au cours de cette étape, la solution du réacteur contenant l'enzyme, les protéines non hydrolysées, les fractions insolubles et les phosphopeptides associés par le calcium, est mise en contact avec une membrane capable de retenir les phosphopeptides tout en laissant passer les peptides non phosphorylés. A cet effet, on utilisera des membranes quelconques organiques ou inorganiques, dont le seuil de coupure est compris entre 2000 et 50000 environ.

L'ultrafiltration associée à l'hydrolyse enzymatique est avantageusement suivie d'une diafiltration qui permet d'épuiser la fraction peptidique non phosphorylée. Au cours de la diafiltration, le produit mis en contact avec la membrane est additionné d'eau ou d'une solution saline convenable. Dans la pratique il s'est avéré que l'ultrafiltrat de lait, préparé au cours d'une opération préalable, ou provenant d'une autre source, peut être avantageusement utilisé comme solution de diafiltration. Pour obtenir de bons résultats, on peut porter le pH de cet ultrafiltrat à des valeurs comprises entre 7 et 8.

Le résultat des opérations précédentes du procédé de l'invention est d'une part une fraction (perméat) comprenant les peptides non phosphorylés, et d'autre part un rétentat contenant les phosphopeptides. La séparation entre les peptides non phosphorylés et les phosphopeptides a été en effet effectuée au sours de l'hydrolyse enzymatique. Les étapes consécutives d'ultrafiltration et de diafiltration permettent de séparer physiquement ces composés.

Dans l'étape suivante du procédé de l'invention, on effectue un traitement de désagrégation des phosphopeptides contenus dans le dernier rétentat. Selon un mode de réalisation pratiquement préféré, on acidifie le rétentat de manière à solubiliser les ions bivalents complexés aux phosphopeptides. A titre d'acide, on peut utiliser n'importe quel acide minéral ou organique compatible avec les buts de la présente invention et la destination des produits. On utilisera donc avantageusement des acides acceptés sur le plan alimentaire. Des exemples d'acides convenables sont l'acide chlorhydrique, l'acide phosphorique, l'acide sulfurique, l'acide lactique, l'acide acétique et autres acides analogues. On a obtenu de bons résultats avec l'acide lactique. On acidifie le rétentat jusqu'à l'obtention d'un pH voisin de 4,6, valeur à laquelle on est certain que la totalité des ions calcium et/ou magnésium a été solubilisée.

En variante, au lieu d'une acidification, on peut réaliser la désagrégation des phosphopeptides par action de complexants du calcium et/ou du magnésium. Au sens de la présente invention, on entend par complexants des composés qui possèdent l'aptitude à fixer le calcium et/ou le magnésium lorsqu'ils sont mis en contact avec les phosphopeptides contenus dans le rétentat. Il s'agit donc en général de substances ayant une action chélatante. Compte tenu de la destination des produits de l'invention, on fera avantageusement appel à des composés acceptés sur le plan alimentaire, en particulier les citrates de sodium ou de potassium.

Lorsqu'on part d'une matière première à base de caséine consistant en du lait écrémé cru ou d'un rétentat d'ultrafiltration d'un tel lait, on a obtenu les meilleurs résultats par acidification du contenu du réacteur enzymatique à l'aide d'acide lactique (à 50 %) jusqu'à un pH de 4,6.

Au cours de la dernière étape du procédé de l'invention, on sépare physiquement une fraction contenant les phosphopeptides en réalisant une dernière ultrafiltration dans des conditions permettant d'obtenir, d'une part, les phosphopeptides et, d'autre part, les sous-produits tels que les résidus pro-téiques et l'enzyme résiduelle. Au cours d'une telle opération il est avantageux que l'ultrafiltration soit

5

suivie d'une diafiltration, opération connue de l'homme de l'art, en vue de la récupération des produits. Dans le cas de la présente étape, une diafiltration à l'eau pure s'est avérée la meilleure.

Les phosphopeptides ainsi obtenus à l'issue du procédé de l'invention constituent le produit de valeur le plus intéressant. Il présente en effet une teneur élevée en phosphosérines et une faible teneur en acides aminés aromatiques (phénylalanine, tyrosine, tryptophane).

La fraction obtenue riche en phosphopeptides peut donc se caractériser par sa composition particulière en acides aminés ainsi que par une teneur élevée en matière minérale (cendres) par rapport à l'azote total, étant donné que la fraction phosphopeptidique contient toute la fraction minérale associée aux caséines de la matière première d'origine (lait ou rétentat de lait).

Le tableau I qui suit définit les caractéristiques essentielles des nouveaux phosphopeptides selon l'invention.

La colonne gauche du tableau I indique les valeurs correspondantes dans le lait, à titre de comparaison.

TABLEAU I.

| | Lait | Peptides non phosphorylés | Phosphopeptides |
|---|---|---|---|
| Somme des acides aminés aromatiques | 12% | >12% | <4% |
| Serine | 5,7% | <4% | <20% et >8% |
| $\dfrac{Ca+Mg+P}{N_T\,(1)}$ | 0,07 | <0,02 | >0,2 |
| Acides aminés libres | — | <10% | <3% |

(1)  $N_T$=azote total×6,38.

Les phosphopeptides obtenus grâce au procédé de l'invention peuvent recevoir de nombreuses applications dans le domaine alimentaire.

Les produits de l'invention (phosphopeptides) sont utiles dans l'alimentation, en particulier dans l'alimentation humaine et dans la nutrition thérapeutique. On sait en effet que, dans le lait humain, le phosphore dit organique, c'est-à-dire lié aux protéines et lié aux lipides, est relativement plus important que dans d'autres laites, notamment le lait de vache. Ainsi, le rapport:

$$\frac{\text{phosphore organique}}{\text{phosphore total}}$$

est d'environ 0,83 dans le lait humain contre 0,34 dans le lait de vache. Plus précisément, le rapport:

$$\frac{\text{phosphore organique lié à l'azote}}{\text{phosphore inorganique}}$$

est d'environ 0,70 dans le lait humain contre 0,36 dans le lait de vache. Les produits de l'invention trouvent donc application dans le domaine connu sous le nom de maternisation des laits.

Mais, d'une façon générale, il est reconnu que la qualité essentielle des protéines du lait de femme est d'assurer un anabolisme azoté remarquable, associé à une charge osmotique rénale et une charge en ions $H^+$ particulièrement faibles.

Or, cette conjonction anabolisme azoté très élevé, charge osmotique rénale et charge en ions $H^+$ faibles, est particulièrement recherchée en réanimation et en nutrition thérapeutique où coexistent très souvent des besoins d'anabolisme élevé et une insuffisance rénale fonctionnelle.

Les produits de l'invention, pour certaines applications, ont une teneur insuffisante en certains acides aminés essentiels (phénylalanine, tyrosine, tryptophane, cystine). Ils peuvant alors avantageusement être associés à d'autres protéines ou peptides ou homologues alpha-céto-acides ou alpha(OH)acides d'acides aminés essentiels, pour que soit rétabli un bon équilibre en acides aminés aboutissant à une valeur biologique optimale.

On notera aussi que de tels produits (phosphopeptides), ont une affinité élevée vis-à-vis des macroéléments (calcium, magnésium) et des oligoéléments tels que, particulièrement, le fer, le zinc, le cuivre, le chrome, le nickel, le cobalt, le manganèse et le sélénium.

**0 033 686**

Les phosphopeptides selon l'invention peuvent être avantageusement transformés en sels desdits éléments par des moyens usuels. Ainsi, pour obtenir un tel sel organophosphoré, on peut utiliser, à titre de solution de diafiltration lors de la purification des phosphopeptides, une solution du sel contenant l'élément à introduire, par exemple une solution de chlorure de fer dans le cas du fer. Ces sels organiphosporés sont très solubles et peuvent avantageusement être utilisés pour véhiculer les éléments en cause. Les produits de l'invention répondent aux besoins nutritionnels des malades souffrant d'insuffisance pancréatique, de maladies métaboliques, d'insuffisance ou de détresse nutritionnelle, associée ou non à une insuffisance rénale fonctionnelle ou organique, en particulier lorsqu'ils sont associés à des peptides, des acides aminés essentiels ou des homologues d'acides aminés essentiels.

L'invention trouve donc une application directe dans des aliments diététiques ou des produits de nutrition thérapeutique parfaitement assimilables par l'organisme humain.

Quelle que soit la source de protéines, de peptides ou d'acides aminés utilisés, ces phosphopeptides permettent de réguler, de la manière la plus souhaitable, le taux de phosphore organique lié à l'azote dans la formule que l'on désire créer.

Ainsi qu'on l'a mentionné précédemment, les phosphopeptides selon l'invention ainsi que leurs dérivés, notamment les sels organophosphorés qu'ils forment avec des éléments minéraux, tels que le calcium ou le magnésium, et/ou avec des oligoéléments (Fe, Zn, Cu, Cr, Ni, Co, Mn, Se par exemple) trouvent une application intéressante en diététique.

L'invention a donc pour objet des compositions diététiques contenant une quantité efficace d'au moins un tel phosphopeptide ou dérivé de phosphopeptide en association avec un véhicule acceptable du point de vue nutritionnel. La quantité efficace peut varier dans de larges limites selon l'effet recherché. A titre indicatif, une quantité de 10 % en poids par rapport au total de la composition convient dans les cas habituels.

Les produits de l'invention peuvent également trouver une application en tant que tels, à titre de médicaments pour l'homme et l'animal.

Les médicaments en cause sont appropriés pour lutter contre toutes les affections impliquant une carence en phosphore organique et en certains éléments minéraux. On donnera ci-après, à titre illustratif et nullement limitatif, des exemples concrets de telles applications.

Les dérivés minéraux des phosphopeptides selon l'invention, consistant en leurs sels de calcium, constituent un supplément minéralo-protidique riche en phosphore organique et en calcium. Ils trouvent une application comme médicaments, par exemple dans les cas suivants:

— recalcification des os après fracture,
— traitement de l'ostéoporose
— supplémentation calcique au cours du traitement du rachitisme.

Les dérivés des phosphopeptides selon l'invention consistant en leurs sels de magnésium constituent eun supplément minéralo-protidique riche en phosphore organique et en magnésium. Ils trouvent une application comme médicaments pour remédier à toutes les formes de déficit magnésique, en particulier chez l'adulte, par exemple dans les cas suivants:

— besoins en Mg fortement accrus par le stress
— mauvaise utilisation du Mg alimentaire chez les vieillards
— augmentation des besoins en Mg chez la femme enceinte.

Des médicaments contenant des dérivés de phosphopeptides consistant en des sels mixtes de calcium et de magnésium sont utilisables de la même manière à titre de supplément minéralo-protidique. Il va sans dire que des applications analogues peuvent être prévues pour des sels variés des phosphopeptides selon l'invention, bien que, dans la pratique, les sels de calcium et/ou magnésium soient préférés.

On notera que les phosphopeptides, tels qu'ils sont obtenus par le procédé de l'invention, sont neutres dans les conditions de pH considérées, par exemple à un pH voisin de 4,6. Dans de telles conditions, en effet, les cations présents dans le milieu, par exemple le calcium et/ou le magnésium, ne sont pas liés aux phosphopeptides. Cependant, dans les conditions d'utilisation, et en particulier lorsque les phosphopeptides sont inclus dans des compositions diététiques ou pharmaceutiques, ils se trouvent sous forme de sels, par exemple de calcium et/ou de magnésium.

Les macroéléments (calcium et/ou magnésium, de préférence) peuvent être remplacés, au moins partiellement, par des oligoéléments.

Les dérivés des phosphopeptides selon l'invention qui contiennent des oligoéléments trouvent une application correspondant à celle du ou des oligoéléments particuliers.

Les indications générales des médicaments contenant des dérivés de phosphopeptides et d'oligoéléments sont notamment les malabsorptions digestives induisant des carences en oligoéléments (Fe, Zn, Cu, Cr, Ni, Mn, Se). Ces malabsorptions digestives apparaissent en particulier lors d'iléites inflammatoires, dans le cas d'intestins réséqués, maladies coeliaques et intestins radiques.

7

**0 033 686**

A titre d'exemples, les carences en zinc peuvent provoquer l'acrodermite entéropathique, des diarrhées, une susceptibilité accrue aux infections, l'hydrogonadisme. Les carences en fer peuvent entraîner de l'anémie sidéropénique.

Les médicaments selon l'invention sont préférés pour le traitement des carences en zinc, cuivre, chrome et fer.

L'invention concerne aussi des compositions pharmaceutiques contenant un produit de l'invention en mélange avec les excipients usuels. Compte tenu de la forme physique du nouveau produit (poudre soluble en milieu aqueux), la forme de présentation ne soulève pas de difficulté. Les nouveaux produits peuvent être ingérés ou administrés en tant que tels, notamment par voie entérale, par exemple en mélange avec l'aliment habituel. Ils peuvent également être présentés sous forme de compositions avec des excipients usuels par exemple convenant à l'administration orale. Des compositions appropriées aux fins de l'invention peuvent ainsi être présentées sous forme de comprimés ou de gélules, avec des excipients connus tels que le talc, le stéarate de magnésium, la silice finement divisée, et tout autre véhicule analogue connu de l'homme de l'art.

A titre d'exemple, on a indiqué ci-après un cas particulier illustrant la préparation de compositions pharmaceutiques pour l'administration par voie orale. Des comprimés one été préparés de la manière usuelle à partir de la formulation suivante:

Phosphopeptide (ou sel de phosphopeptide) selon l'invention          200 mg

excipient QS pour un comprimé terminé à          300 mg

L'excipient utilisé peut être le talc, le stérate de magnésium ou la silice disponible sur le marché sous la dénomination "aérosil".

On a préparé de la même manière des gélules dosées à 100 mg de phosphopeptide (ou de sel) selon l'invention et contenant un excipient usuel QS pour une gélule terminée à 200 mg.

L'invention sera encore illustrée sans être aucunement limitée par la description qui suit et des exemples concrets de réalisation.

La description ci-après est faite en référence aux dessins annexés sur lesquels:

Fig. 1 représente un schéma de réalisation du procédé de l'invention appliqué à une matière première à base de caséine consistant en du lait;

Fig. 2 est un schéma analogue à celui de la Figure 1, illustrant l'application du procédé de l'invention à un rétentat d'ultrafiltration de lait;

Fig. 3 représente d'une manière schématique le réacteur enzymatique à membrane susceptible d'être mis en oeuvre pour les besoins de l'invention.

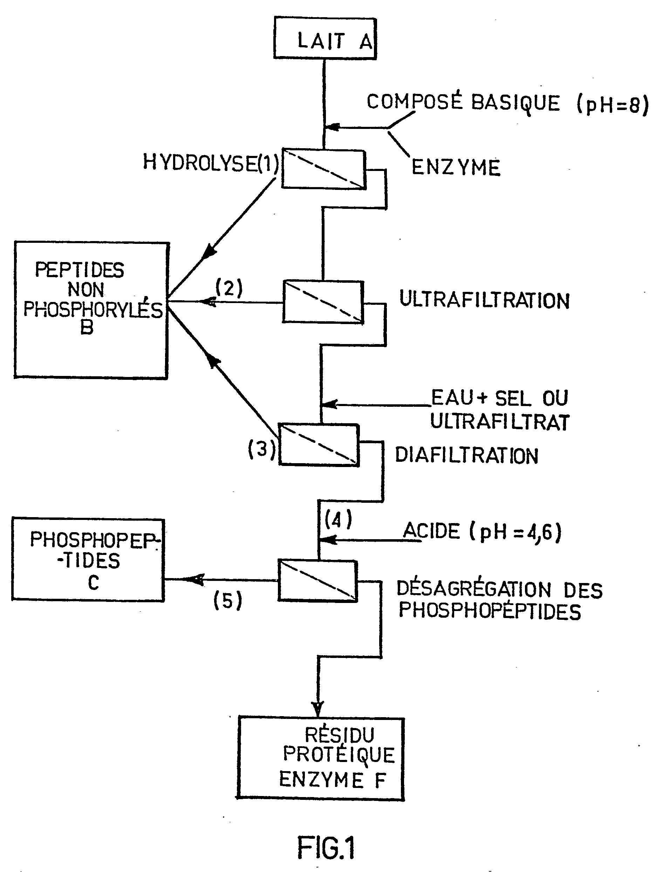

La Figure 1 est un schéma de réalisation du procédé de l'invention appliqué au traitement d'une matière première à base de caséine consistant en du lait cru écrémé. Cette matière première est désignée par la référence lait A. Conformément à l'invention, le lait est soumis à une réaction d'hydrolyse enzymatique en présence d'au moins une enzyme protéolytique, telle que la pancréatine, en milieu basique, par exemple à un pH voisin de 8. La température est avantageusement de 37°C. On a matérialisé l'étape d'hydrolyse en réacteur enzymatique par la référence 1.

A la fin de l'hydrolyse, la solution du réacteur contenant l'enzyme, les protéines non hydrolysées, les fractions insolubles et les phosphopeptides associés au calcium, a été concentrée par ultrafiltration (référence 2). Consécutivement, on a réalisé une diafiltration en mettant en contact le rétentat issu de l'ultrafiltration 2 avec un module d'ultrafiltration, tout en l'additionnant en continu d'un ultrafiltrat de lait ajusté à pH 8. L'opération de diafiltration est matérialisée au dessin par la référence 3.

Les ultrafiltrats respectifs des opérations 1, 2 et 3 ci-dessus sont rassemblés et constituent ensemble une fraction consistant en des peptides non phosphorylés (référence B).

Le rétentat provenant de la diafiltration 3 est d'abord soumis à une désagrégation des phosphopeptides qu'il contient, avantageusement par acidification à pH 4,6, en particulier avec de l'acide lactique. Cette opération est matérialisée par la référence 4. Ensuite, on a représenté par la référence 5 l'étape finale d'ultrafiltration qui comporte aussi une diafiltration à l'eau et à l'issue de laquelle on sépare, d'une part, une fraction C de phosphopeptides et, d'autre part, une fraction F contenant l'enzyme résiduelle ainsi que le résidu protéique.

En partant de 1000 litres de lait, conformément au schéma réactionnel de la Figure 1, on peut obtenir 900 à 950 litres de solution peptidique à 27 g/litre et environ 50 litres de phosphopeptides à 80 g/litre.

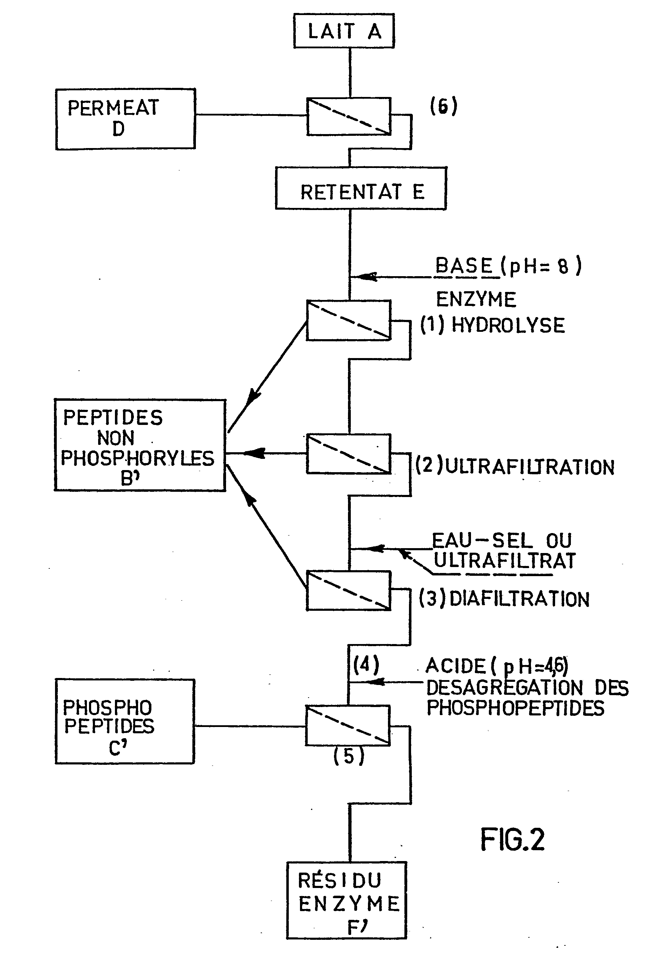

Dans la variante de l'invention représentée à la Figure 2, on soumet tout d'abord le lait A cru et écrémé à une ultrafiltration (référence 6) jusqu'à un taux de concentration voisin de 2. On sépare ainsi un perméat D et en rétentat E. C'est le rétentat qui sert alors de matière première à base de caséine. Le rétentat E est amené à pH 8 par addition d'un composé basique et hydrolysé en réacteur enzymatique en présence d'une enzyme protéolytique (par exemple avec 4 g/litre d'enzyme pancréatique à une température de 37°C). Comme sur la Figure 1, 1'-étape d'hydrolyse est matérialisée par la référence 1.

En fin d'hydrolyse, la solution résiduelle du réacteur enzymatique a été concentrée par ultrafiltration (référence 2) et diafiltrée (référence 3) avec de l'ultrafiltrat à pH 7,0 afin d'épuiser la fraction peptidique

8

**0 033 686**

non phosphorylée. Les divers ultrafiltrats des ultrafiltrations 1, 2 et 3 sont rasemmblés et constituent la fraction peptidique non phosphorylée B'.

De même qu'à la Figure 1, le contenu du réacteur subit un traitement destiné à désagréger les phosphopeptides, avantageusement une acidification jusqu'à un pH de 4,6 par exemple par addition d'acide lactique à 50 % (référence 4). La solution contenue dans le réacteur est alors soumise à une ultrafiltration et une diafiltration à l'eau, matérialisées par la référence 5, ce qui permet de séparer les phosphopeptides (C') et une fraction résiduelle protéique-enzyme (F').

En travaillant conformément au schéma réactionnel de la Figure 2, et en partant de 1000 litres de lait, on peut obtenir 400 à 450 litres de solution peptidique à 57 g/litre et environ 50 litres de solution de phosphopeptides à 80 g/litre.

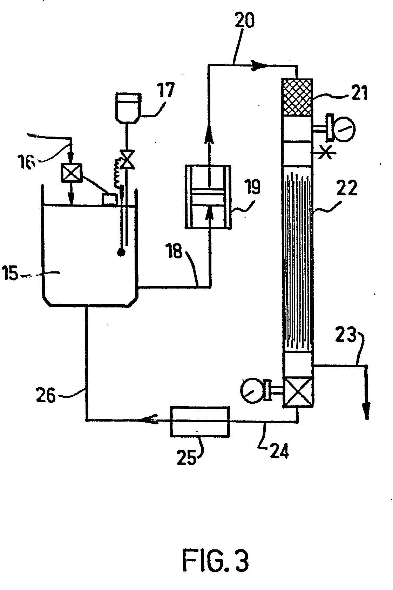

La Figure 3 représente un réacteur enzymatique à membrane qui peut être utilisé pour le procédé de l'invention.

Celui-ci comporte d'abord une cuve à réaction désignée par la référence générale 15. L'alimentation continue en phosphocaséinates se fait par la canalisation 16. Un appareil 17 sert à la fois à mesurer et à maintenir le pH dans la cuve de réaction en neutralisant les ions $H^+$ libérés lors de la coupure des liaisons peptidiques. L'appareil était un pH–stat Mettler comprenant un amplificateur de potential, un présélecteur du point d'équivalence et une burette automatique de distribution du réactif, ce fernier étant un composé basique tel que mentionné ci-dessus. Aucun encrassement excessif de l'électrode n'a été constaté. Le produit d'hydrolyse prélevé dans la cuve de réaction par la canalisation 18 est acheminé par une pompe pneumatique 19 à membrane. Un exemple pratique est la pompe modèle Amicon LP 20 A, capable de débiter 720 l/h à 1,7 bar. Au refoulement de la pompe, le produit passe dans une canalisation 20 et est amené sur un préfiltre 21 ayant une porosité de 150 microns. La référence 22 désigne le module d'ultrafiltration. Dans un exemple spécifique, on a utilisé le système DC 10 S de la Société Amicon comprenant des cartouches d'ultrafiltration à fibres creuses. Le perméat était récupéré dans une canalisation 23 et constituait l'hydrolysat peptidique recherché. Le rétentat du module 22 était prélevé par la canalisation 24, introduit sur un échangeur 25 et acheminé par la canalisation 26 pour être recyclé dans la cuve de réaction 15.

Les membranes utilisées étaient du type fibres creuses ayant les caractéristiques suivantes:

TABLEAU II

| Type | Seuil de coupure | Surface | Fabricant |
|---|---|---|---|
| H 10 P 5 | 5.000 | 0,9 m$^2$ | Amicon |
| H 10 P 10 | 10.000 | | |
| PM 2 | 2.000 | 1,4 m$^2$ | Romicon |
| PM 50 | 50.000 | | |

Exemple 1

Cet exemple est relatif à l'utilisation de lait à titre de matière première.

Le lait cru était, après écrémage, hydrolysé dans un réacteur enzymatique identique à celui représenté sur la Figure 3; les membranes utilisées étaient du type XM 50 (fibres creuses). L'enzyme (pancréatine d'origine bovine d'activité 4 NF) provenait de Sigma et sa concentration était de 2 g/litre. Le pH du réacteur était maintenu à 8 par addition d'hydroxyde de sodium 2 N. L'hydrolyse était conduite à 37°C.

L'ultrafiltrat recueilli au cours de l'hydrolyse constituait la fraction qualifiée de peptides non phosphorylés. En fin d'hydrolyse, le contenu du réacteur étain concentré 3,4 fois afin de diminuer le volume à diafiltrer. La diafiltration a été réalisée avec de l'ultrafiltrat obtenu par traitement du lait sur membrane XM 50 à 25°C et amené à pH 8 avec de la soude 2N. La diafiltration avait pour but d'épuiser le contenu du réacteur en peptides non phosphorylés. La solution résiduelle du réacteur enzymatique contenant l'enzyme et les phosphopeptides agglomérés était alors acidifiée jusqu'à pH 4,6 par addition d'acide lactique à 50 %. Les phosphopeptides dissociés étaient recueillis dans le perméat.

Les analyses chimiques des différents produits obtenus au cours de cette fabrication sont rassemblées dans les tableaux III et IV suivants.

9

**0 033 686**

TABLEAU III
Composition chimique (g/kg)

| | E.S.* | $N_T$** | Ca | P |
|---|---|---|---|---|
| Lait départ | 90,0 | 29,3 | 1,20 | 1,02 |
| Contenu du réacteur en fin d'hydrolyse | 88,5 | 31,2 | 1,20 | 1,02 |
| Contenu du réacteur concentré par ultrafiltration | 112,4 | 40,7 | 3,81 | 2,65 |
| Contenu du réacteur après diafiltration | 87,0 | 14,4 | 4,17 | 3,08 |
| Peptides non phosphorylés | 80,0 | 27,0 | 0,14 | 0,35 |
| Phosphopeptides | 89,0 | 8,2 | 3,40 | 2,40 |

* E.S.: extrait sec     ** $N_T$=azote total$\times$6,38

TABLEAU IV
Composition en acides aminés.

| | Peptides non phosphorylés | Phospho-peptides | | Peptides non phosphorylés | Phospho-peptides |
|---|---|---|---|---|---|
| Asp | 6,92 | 9,22 | Cys | 0,68 | $\varepsilon$ |
| Thr | 3,55 | 4,50 | Met | 2,01 | 0,58 |
| Ser | 3,58 | 11,80 | Ile | 4,42 | 7,58 |
| Pro | 9,07 | 5,88 | Leu | 8,92 | 4,95 |
| Glu | 19,40 | 30,80 | Tyr | 4,92 | <2 |
| Gly | 1,71 | 2,38 | Phe | 4,46 | 1,80 |
| Ala | 2,83 | 2,96 | Lys | 7,56 | 4,57 |
| Val | 5,57 | 7,12 | His | 2,60 | 1,37 |
| | | | Arg | 3,09 | 1,96 |

Exemple 2

Cet exemple est relatif à l'utilisation de rétentat d'ultrafiltration du lait, à titre de matière première.

Le lait cru était, après écrémage, concentré 2 fois par ultrafiltration à 25°C sur membrane XM 50. Le pH du rétentat était alors ajusté à 8 par addition d'hydroxyde de sodium 2N. Le rétentat était ensuite hydrolysé dans le réacteur enzymatique (Figure 3). La nature de l'enzyme était identique à celle utilisée à l'exemple 1 et sa concentration était de 4 g/litre. L'hydrolyse était conduite à 37°C et le pH de la solution était maintenu à 8 par addition d'hydroxyde de sodium 2N.

L'ultrafiltration recueilli au cours de l'hydrolyse constituait la fraction qualifiée de peptides non phosphorylés. En fin d'hydrolyse, le contenu du réacteur enzymatique était concentré 3 fois par ultrafiltration; la diafiltration a été effectuée dans ce cas avec de l'ultrafiltrat de lait à pH 7 afin d'épuiser la solution de réacteur en peptides non phosphorylés. Le contenu du réacteur était alors acidifié à pH 4,6 avec de l'acide lactique à 50 %. Les phosphopeptides dissociés étaient alors recueillis dans le perméat.

Les analyses chimiques et la composition en acides aminés des différents produits obtenus figurent dans les tableaux V et VI ci-après.

Des exemples illustratifs concernant l'application des produits de l'invention seront indiqués ci'après, à titre nullement limitatif.

10

# 0 033 686

## TABLEAU V
### Composition chimique (g/kg)

|  | E.S. | $N_T$ | Ca | P |
|---|---|---|---|---|
| Lait | 90,0 | 30,0 | 1,15 | 1,00 |
| Rétentat | 122,9 | 59,8 | 1,90 | 1,63 |
| Contenu du réacteur en fin d'hydrolyse après ultrafiltration | 148,5 | 73,5 | 5,09 | 3,97 |
| Contenu du réacteur après diafiltration | 80,0 | 14,34 | 3,39 | 2,70 |
| Peptides non phosphorylés | 104,0 | 53,3 | 0,20 | 0,40 |
| Phosphopeptides | 74,3 | 7,5 | 2,55 | 1,69 |

## TABLEAU VI
### Compositions en acides aminés

|  | Peptides non phosphorylés | Phospho-peptides |  | Peptides non phosphorylés | Phospho-peptides |
|---|---|---|---|---|---|
| Asp | 7,32 | 8,02 | Cys | 0,66 | $\varepsilon$ |
| Thr | 4,56 | 4,42 | Met | 2,44 | 1,49 |
| Ser | 3,97 | 11,98 | Ile | 4,73 | 7,71 |
| Pro | 9,30 | 5,29 | Leu | 9,70 | 5,17 |
| Glu | 20,61 | 26,50 | Tyr | 5,20 | 1,25 |
| Gly | 1,88 | 2,73 | Phe | 4,88 | 1,80 |
| Ala | 3,16 | 3,15 | Lys | 7,79 | 4,90 |
| Val | 5,95 | 7,42 | His | 2,84 | 1,47 |
|  |  |  | Arg | 3,74 | 2,10 |

Exemple 3

Cet exemple concerne un produit de réanimation destiné à un usage par voie entérale chez des malades nécessitant un apport protéique de l'ordre de 7 à 15 % de l'Apport Calorique Total (ACT), par exemple dans les affections suivantes:mucoviscidose ou fibrose kystique du pancréas, insuffisance rénale, malades présentant une atteinte infectieuse ou inflammatoire de la paroi intestinale, détresse nutritionnelle exigeant un anabolisme intense, une charge rénale osmotique et une charge en ions $H^+$ réduites. Ces protéines sont amenées de préférence sous forme pré-digérée.

Exemple de formule centésimale:
— mélange de petits peptides selon l'invention:

| | | |
|---|---|---|
| . Phosphopeptides | de 4 à 8 % | ⎫ |
| . Peptides de lactosérum | 60 % | ⎬ 2,50 g |
| . Peptides de caséine | de 32 à 36 % | ⎪ |
| . CMP | de 0 à 4 % | ⎭ |

**0 033 686**

— lipides:
mélange à parties égales de:
. Huile de beurre    0,5 g
. Triglycérides à chaîne moyenne (TCM)    0,5 g
. Huile de maïs    0,5 g     4,10 g
. Huile de tournesol    0,5 g
Monostéarate de glycérol    2,1 g

— glucides:
. Polymères de glucose    10 g
. Glucose    1,5 g     13,00 g
. Galactose    1,5 g

— vitamines:
A, D, E, $B_1$, $B_2$, PP, $B_5$, $B_6$, $B_{12}$, acide    selon les
folique Biotine, Vit. C    recommandations
FAO/OMS

— éléments minéraux:
(calcium, sodium, potassium, magnésium, phosphore,
zinc, fer, cuivre, manganèse, chlore, iode)     0,455 g

— eau distillée     q.s.p. 100

Exemple 4

Cet exemple concerne un produit de réanimation destiné à un usage par voie entérale chez des malades nécessitant un apport protéique de l'ordre de 12 à 25 % de l'apport Calorique Total, sous forme de protéines. Une telle alimentation est appropriée à toute situation nécessitant un anabolisme azoté important et une surcharge en $P^-$ organique.

Exemple de formule centésimale:
— mélange de petits peptides selon l'invention
. Phosphopeptides    de 8 à 28 %
. Peptides de lactosérum    de 22 à 60 %     de 3 à 6,25 g
. Peptides de caséine    de 12 à 70 %

— lipides:
. T.C.M.    2,30 g
. Huile très riche en acides gras essentiels    0,50 g     2,90 g
. Emulsifiant    0,10 g

— glucides:
. Petits polymères de glucose    de 8,25 à 10,5 g
. Glucose    de 2 à 2,5 g     de 12,25 g
. Galactose    de 2 à 2,5 g     15,5 g

— vitamines:
A, D, E, $B_1$, $B_2$, PP, $B_5$, $B_6$, $B_{12}$, acide    selon les
folique, Biothine Vitamine C    recommandations
FAO/OMS

— éléments minéraux:
(calcium, sodium, potassium, magnésium, phosphore,
zinc, fer, cuivre, manganèse, chlore, iode)     0,455 g

— eau distillée     q.s.p. 100

Exemple 5

Cet exemple concerne un produit de réanimation destiné à un usage par voie entérale chez des malades nécessitant un apport protéique de l'ordre de 7 à 12 % de l'A.C.T., notamment dans les affections suivantes:mucoviscidose ou fibrose kystique du pancréas, insuffisance rénale, malades présentant une atteinte infectieuse ou inflammatoire de la paroi intestinale, détresse nutritionnelle exigeant un anabolisme intense et une surcharge rénale osmotique, une surcharge en ions $H^+$ réduites. Ces protéines sont amenées de préférence sous forme prédigérée.

12

**0 033 686**

Exemple de formule centésimale:
— Mélange de petits peptides selon l'invention:

| | | |
|---|---|---|
| . Phosphopeptides | 4 % | |
| . Peptides de caséine | 36 % | 2,50 g |
| . Peptides de lactosérum | 40 % | |
| . CMP | 20 % | |

— lipides:
mélange à parties égales de:

| | | |
|---|---|---|
| . Huile de beurre | 0,5 g | |
| . TCM | 0,5 g | |
| . Huile de maïs | 0,5 g | 4,10 g |
| . Huile de tournesol | 0,5 g | |
| . Monostéarate de glycérol | 2,1 g | |

— glucides:

| | | |
|---|---|---|
| . Polymères de glucose | 10 g | |
| . Glucose | 1,5 g | 13,00 g |
| . Galactose | 1,5 g | |

— vitamines:

A, D, E, $B_1$, $B_2$, PP, $B_5$, $B_6$, $B_{12}$, acide folique, Biotine, Vit. C — selon les recommandations FAO/OMS

— éléments minéraux:

(calcium, sodium, potassium, magnésium, phosphore, zinc, fer, cuivre, manganèse, chlore, iode) — 0,455 g

— eau distillée — q.s.p. 100

Le mélange de peptides contenant les phosphopeptides de l'invention peut avantageusement comprendre les peptides obtenus conformément au procédé décrit dans la demande de brevet français déposée le 26 Juin 1979 sous le No. 79 16 483 (publication: FR—A—2 459 620) au nom de l'Institut National de la Recherche Agronomique pour "Hydrolysat enzymatique total de protéines de lactosérum, obtention et applications". On rappellera qu'un tel hydrolysat peptidique ne contient pratiquement pas de protéines résiduelles et que 50 % des peptides contiennent 2 à 5 acides aminés. Plus particulièrement, l'hydrolysat contient 7 à 90 % de l'azote présent sous forme de peptides ayant un nombre d'acides aminés inférieur à 10. Sous une forme spécifique, l'hydrolysat répond à l'amino-gramme ci-après:

Aminogramme:

| | | | |
|---|---|---|---|
| Ile | 6,0 | —Arg | 2,7 |
| Leu | 9,9 | —His | 1,7 |
| Lys | 9,2 | —Ala | 4,9 |
| Cys | 1,8 | —Asp | 9,5 |
| Phe | 3,2 | —Glu | 17,6 |
| Thr | 6,7 | —Gly | 1,7 |
| Tyr | 3,6 | —Pro | 6,2 |
| Trp | 2,0 | —Ser | 5,5 |
| Val | 5,5 | —Met | 2,0 |

Le procédé pour l'obtention d'un tel hydrolysat consiste à effectuer d'abord l'ultrafiltration du lactosérum puis son hydrolyse enzymatique et il est plus particulièrement remarquable en ce qu'on met en contact le rétentat d'ultrafiltration avec une enzyme protéolytique capable de reconstituer la

. 13

**0 033 686**

digestion protéique qui prend place in vivo dans l'organisme humain, ladite enzyme étant de préférence la pancréatine, l'hydrolyse étant conduite jusqu'à ce que le produit ne contienne plus de protéines résiduelles, c'est-à-dire ne présente aucun azote précipitable par l'acide trichloroacétique à 12 %, après quoi on récupère l'hydrolysat obtenu qui représente l'hydrolysat enzymatique total recherché.

**Revendications pour les Etats contractants: BE, CH, DE, FR, GB, IT, LI, LU, NL, SE**

1. Procédé pour le traitement d'une matière première à base de caséine contenant des phosphocaséinates de cations bivalents, tels que les phosphocaséinates de calcium et/ou de magnésium, caractérisé en ce qu'on soumet la matière première à une hydrolyse enzymatique à l'aide d'au moins une enzyme protéolytique capable de reconstituer la digestion protéique qui prend place in vivo dans l'organisme humain, en ce qu'on récupère l'hydrolysat obtenu, en ce qu'on soumet celui-ci à au moins une ultrafiltration sur des membranes capables de retenir les phosphopeptides, tout en laissant passer les peptides, l'ultrafiltrat content ainsi les peptides non phosphorylés, en ce qu'on récupère le rétentat d'ultrafiltration, en ce qu'on effectue en traitement de désagrégation des phosphopeptides contenus dans le rétentat, et en ce qu'on soumet celui-ci à au moins une nouvelle ultrafiltration sur des membranes ne retenant pas les phosphopeptides, lesquels sont ainsi séparés de l'enzyme et peuvent être récupérés en tant que produit.

2. Procédé selon la revendication 1, caractérisé en ce que, à titre de matière première à base de caséine, on utilise du lait, un rétentat d'ultrafiltration du lait, ou encore des phosphocaséinates de cations bivalents isolés au préalable, tels que des phosphocaséinates d'alcalino-terreux, par exemple de calcium ou de magnésium.

3. Procédé selon la revendication 2, caractérisé en ce qu'on utilise comme matière première un rétentat obtenu par ultrafiltration de lait, à un taux de concentration de 1,5 à 3, par exemple de 2 environ, cette ultrafiltration pouvant être suivie d'une diafiltration.

4. Procédé selon l'une quelconque des revendications 1 à 3, caractérisé en ce qu'on effectue l'hydrolyse enzymatique dans un dispositif qui combine un équipement d'ultrafiltration à un réacteur enzymatique, constituant ainsi un réacteur enzymatique à membrane.

5. Procédé selon la revendication 4, caractérisé en ce qu'on réalise en continu l'hydrolyse enzymatique en introduisant la matière première à base de caséine dans une zone de réaction en vue d'une mise en contact intime avec l'enzyme, on soutire en continu le produit de réaction en l'amenant de la zone de réaction dans une zone d'ultrafiltration, de laquelle on soutire également en continu un perméat qui constitue l'hydrolysat peptidique.

6. Procédé selon l'une quelconque des revendications 1 à 5, caractérisé en ce qu'on ajuste le pH entre 7 et 9, de préférence entre 7 et 8,5, par exemple à 8 environ, au cours de l'hydrolyse enzymatique.

7. Procédé selon l'une quelconque des revendications 1 à 6, caractérisé en ce qu'à titre d'enzyme, on utilise une pancréatine sous forme d'extrait pancréatique naturel, avec des mélanges synthétiques de trypsine et d'alphachymotrypsine.

8. Procédé selon l'une quelconque des revendications 1 à 7, caractérisé en ce que la température d'hydrolyse est choisi entre 37 et 45°C, de préférence entre 37 et 40°C et, avantageusement, de l'ordre de 37°C.

9. Procédé selon l'une quelconque des revendications 1 à 8, caractérisé en ce que, dans le réacteur enzymatique à membrane, on utilise des membranes capables de retenir efficacement l'enzyme.

10. Procédé selon l'une quelconque des revendications 1 à 9, caractérisé en ce qu'après l'hydrolyse, la solution résiduelle du réacteur enzymatique est soumise à une ultrafiltration sur des membranes capables de retenir les phosphopeptides, tout en laissant passer les peptides non phosphorylés, par exemple des membranes dont le seuil de coupure est compris entre 2000 et 50.000 environ.

11. Procédé selon la revendication 10, caractérisé en ce qu'on fait suivre l'ultrafiltration d'une diafiltration en vie d'épuiser la fraction peptidique non phosphorylée en additionnant le produit, mis en contact avec la membrane, d'eau ou d'une solution saline et, avantageusement, d'un ultrafiltrat de lait porté à un pH entre 7 et 8.

12. Procédé selon l'une quelconque des revendications 1 à 11, caractérisé en ce que le traitement de désagrégation des phosphopeptides contenus dans le rétentat est effectué par acidification, par exemple jusqu'à un pH voisin de 4,6, à l'aide d'un acide minéral ou organique, de préférence accepté sur le plan alimentaire, avantageusement l'acide lactique.

13. Procédé selon l'une quelconque des revendications 1 à 11, caractérisé en ce que le traitement de désagrégation des phosphopeptides contenus dans le rétentat est effectué par action de complexants du calcium et/ou du magnésium, tels que des citrates.

14. Procédé selon l'une quelconque des revendications 1 à 13, caractérisé en ce qu'au cours de la dernière étape du procédé, on sépare physiquement, par ultrafiltration, une fraction contenant les phosphopeptides désirés, des résidus protéiques et de l'enzyme résiduelle.

15. Procédé selon l'une quelconque des revendications 1 à 14, caractérisé en ce qu'on transforme les phosphopeptides en sels de phosphopeptides et de macroéléments, tels que le calcium et/ou le magnésium, par mise en présence desdits phosphopeptides avec des cations ou des mélanges de cations desdits macroéléments, ce qui conduit à des sels organophosphorés desdits macroéléments.

16. Procédé selon la revendication 15, caractérisé en ce qu'on remplace au moins partiellement les macroéléments par des oligoéléments, tels que le fer, le zinc, le cuivre, le chrome, le nickel, le cobalt, le manganèse et le sélénium, en utilisant, lors de la purification des phosphopeptides une solution contenant le ou les oligoéléments à introduire, ce qui conduit à des sels organophosphorés avec des oligoéléments liés.

17. Sels organophosphorés de phosphopeptides et d'éléments minéraux, caractérisés par les paramètres ci-après:

Somme des acides aminés aromatiques <4 %

$$\text{rapport } \frac{Ca+Mg+P}{N_T} > 0,2$$

Taux de sérines supérieur teneur en acides aminés à 8% et inférieur à 20 %; libres <3 %.

18. Aliments, ou compléments alimentaires, répondant à des besoins spécifiques nutritionnels et contenant un produit selon la revendication 17 ou un produit résultant du procédé selon l'une quelconque des revendications 1 à 16.

19. Aliments diététiques ou de nutrition thérapeutiques contenant un produit selon la revendication 17 ou un produit résultant du procédé selon l'une quelconque des revendications 1 à 16, en association éventuelle avec un véhicule neutre convenant à l'alimentation orale ou entérale.

20. Aliments selon la revendication 19 contenant en outre des sources complémentaires d'acides aminés essentiels.

21. Médicaments contenant un produit selon la revendication 17 ou un produit résultant du procédé selon l'une quelconque des revendications 1 à 16.

22. Composition pharmaceutique contenant une quantité efficace d'au moins un produit selon la revendication 17 ou d'un produit résultant du procédé selon l'une quelconque des revendications 1 à 16, en association avec un véhicule pharmaceutiquement acceptable, convenant notamment à l'administration entérale.

23. Composition selon la revendication 22, contenant au moins un sel de phosphopeptide selon la revendication 17 ou résultant du procédé selon la revendication 15, en particulier de calcium et/ou de magnésium, constituant un supplément minéralo-protodique riche en phosphore organique, en calcium et/ou magnésium.

24. Composition selon la revendication 22, contenant au moins un sel de phosphopeptide résultant du procédé selon la revendication 16, pour remédier aux malabsorptions digestives induisant des carences en oligoéléments.

**Revendications pour l'Etat contractant: AT**

1. Procédé pour le traitement d'une matière première à base de caséine contenant des phosphocaséinates de cations bivalents, tels que les phosphocaséinates de calcium et/ou de magnésium, caractérisé en ce qu'on soumet la matière première à une hydrolyse enzymatique à l'aide d'au moins une enzyme protéolytique capable de reconstituer la digestion protéique qui prend place in vivo dans l'organisme humain, en ce qu'on récupère l'hydrolysat obtenu, en ce qu'on soumet celui-ci à au moins une ultrafiltration sur des membranes capables de retenir les phosphopeptides, tout en laissant passer les peptides, l'ultrafiltrat contenant ainsi les peptides non phosphorylés, en ce qu'on récupère le rétentat d'ultrafiltration, en ce qu'on effectue un traitement de désagrégation des phosphopeptides contenus dans le rétentat, et en ce qu'on soumet celui-ci à au moins une nouvelle ultrafiltration sur des membranes ne retenant pas les phosphopeptides, lesquels sont ainsi séparés de l'enzyme et peuvent être récupérés en tant que produit.

2. Procédé selon la revendication 1, caractérisé en ce que, à titre de matière première à base de caséine, on utilise du lait, un rétentat d'ultrafiltration du lait, ou encore des phosphocaséinates de cations bivalents isolés au préalable, tels que des phosphocaséinates d'alcalinoterreux, par exemple de calcium ou de magnésium.

3. Procédé selon la revendication 2, caractérisé en ce qu'on utilise comme matière première un rétentat obtenu par ultrafiltration de lait, à un taux de concentration de 1,5 à 3, par exemple de 2 environ, cette ultrafiltration pouvant être suivie d'une diafiltration.

4. Procédé selon l'une quelconque des revendications 1 à 3, caractérisé en ce qu'on effectue l'hydrolyse enzymatique dans un dispositif qui combine un équipement d'ultrafiltration à un réacteur enzymatique, constituant ainsi un réacteur enzymatique à membrane.

5. Procédé selon la revendication 4, caractérisé en ce qu'on réalise en continu l'hydrolyse

**0 033 686**

enzymatique en introduisant la matière première à base de caséine dans une zone de réaction en vue d'une mise en contact intime avec l'enzyme, on soutire en continu le produit de réaction en l'amenant de la zone de réaction dans une zone d'ultrafiltration, de laquelle on soutire également en continu un perméat qui constitue l'hydrolysat peptidique.

6. Procédé selon l'une quelconque des revendications 1 à 5, caractérisé en ce qu'on ajuste le pH entre 7 et 9, de préférence entre 7 et 8,5, par exemple à 8 environ, au cours de l'hydrolyse enzymatique.

7. Procédé selon l'une quelconque des revendications 1 à 6, caractérisé en ce qu'à titre d'enzyme, on utilise une pancréatine sous forme d'extrait pancréatique naturel, ou des mélanges synthétiques de trypsine et d'alphachymotrypsine.

8. Procédé selon l'une quelconque des revendications 1 à 7, caractérisé en ce que la température d'hydrolyse est choisie entre 37 et 45°C, de préférence entre 37 et 40°C et, avantageusement, de l'ordre de 37°C.

9. Procédé selon l'une quelconque des revendications 1 à 8, caractérisé en ce que, dans le réacteur enzymatique à membrane, on utilise des membranes capables de retenir efficacement l'enzyme.

10. Procédé selon l'une quelconque des revendications 1 à 9, caractérisé en ce qu'après l'hydrolyse, la solution résiduelle du réacteur enzymatique est soumise à une ultrafiltration sur des membranes capables de retenir les phosphopeptides, tout en laissant passer les peptides non phosphorylés, par exemple des membranes dont le seuil de coupure est compris entre 2000 et 50.000 environ.

11. Procédé selon la revendication 10, caractérisé en ce qu'on fait suivre l'ultrafiltration d'une diafiltration en vue d'épuiser la fraction peptidique non phosphorylée en additionnant le produit, mis en contact avec la membrane, d'eau ou d'une solution saline et, avantageusement, d'un ultrafiltrat de lait porté à un pH entre 7 et 8.

12. Procédé selon l'une quelconque des revendications 1 à 11, caractérisé en ce que le traitement de désagrégation des phosphopeptides contenus dans le rétentat est effectué par acidification, par exemple jusqu'à un pH voisin de 4,6, à l'aide d'un acide minéral ou organique, de préférence accepté sur le plan alimentaire, avantageusement l'acide lactique.

13. Procédé selon l'une quelconque des revendications 1 à 11, caractérisé en ce que le traitement de désagrégation des phosphopeptides contenus dans le rétentat est effectué par action de complexants du calcium et/ou du magnésium, tels que des citrates.

14. Procédé selon l'une quelconque des revendications 1 à 13, caractérisé en ce qu'au cours de la dernière étape du procédé, on sépare physiquement, par ultrafiltration, une fraction contenant les phosphopeptides désirés, des résidus protéiques et de l'enzyme résiduelle.

15. Procédé selon l'une quelconque des revendications 1 à 14, caractérisé en ce qu'on transforme les phosphopeptides en sels de phosphopeptides et de macroéléments, tels que le calcium et/ou le magnésium, par mise en présence desdits phosphopeptides avec des cations ou des mélanges de cations desdits macroéléments, ce qui conduit à des sels organophosphorés desdits macroéléments.

16. Procédé selon la revendication 15, caractérisé en ce qu'on remplace au moins partiellement les macroéléments par des oligoéléments, tels que le fer, le zinc, le cuivre, le chrome, le nickel, le cobalt, le manganèse et le sélénium, en utilisant, lors de la purification des phosphopeptides une solution contenant le ou les oligoéléments à introduire, ce qui conduit à des sels organophosphorés avec des oligoéléments liés.

17. Procédé selon l'une quelconque des revendications précédentes, caractérisé en ce que l'on prépare des sels organophosporés de phosphopeptides et d'éléments minéraux caractérisés par les paramètres ci-après:

somme des acides aminés aromatiques <4%

$$\text{rapport } \frac{Ca+Mg+P}{N_T} > 0{,}2$$

taux de sérines supérieur à 8% et inférieur à 20%, teneur en acides aminés libres <3%.

18. Procédé de préparation d'aliments ou compléments alimentaires, répondant à des besoins spécifiques nutritionnels caractérisé en ce qu'on y inclut un produit résultant du procédé selon l'une quelconque des revendications 1 à 17.

19. Procédé de préparation d'aliments diététiques ou de nutrition thérapeutiques contenant un produit préparé selon l'une des revendications 1 à 18, en association éventuelle avec un véhicule neutre convenant à l'alimentation orale ou entérale.

20. Procédé de préparation d'aliments préparés selon la revendication 19 contenant en outre des sources complémentaires d'acides aminés essentiels.

21. Procédé de préparation d'une composition pharmaceutique contenant une quantité efficace

16

# 0 033 686

d'au moins un produit résultant du procédé selon l'une quelconque des revendications 1 à 17, caractérisé en ce que on associe ledit produit avec un véhicule pharmaceutiquement acceptable, convenant notamment à l'administration entérale.

22. Procédé de préparation d'une composition selon la revendication 21, contenant au moins un sel de phosphopeptide, en particulier de calcium et/ou de magnésium, constituant un supplément minéralo-protidique riche en phosphore organique en calcium et/ou magnésium, caractérisé en ce que ledit sel est préparé selon l'une des revendications 15 ou 17.

**Patentansprüche für die Vertragsstaaten: BE, CH, DE, FR, GB, IT, LI, LU, NL, SE**

1. Verfahren zur Behandlung eines Rohmaterials auf der Basis von Casein, das Phosphocaseinate von zweiwertigen Kationen, z.B. Phosphocaseinate von Calcium und/oder Magnesium, enthält, dadurch gekennzeichnet, daß man das Rohmaterial einer enzymatischen Hydrolyse mit Hilfe wenigstens eines proteolytischen Enzyms unterwirft, das die in vivo im menschlichen Organismus stattfindende Proteinverdauung wieder zu erzeugen vermag, daß man das erhaltene Hydrolysat gewinnt, indem man dieses wenigstens einer Ultrafiltration auf Membranen unterwirft, die die Phosphopeptide zurückzuhalten vermögen, während sie die Peptide durchgehen lassen, so daß das Ultrafiltrat in dieser Weise die nicht phosphorylierten Peptide enthält, daß man den Ultrafiltrationsrückstand gewinnt, daß man eine Zerfallsbehandlung der im Rückstand enthaltenen Phosphopeptide vornimmt und daß man den Rückstand wenigstens einer erneuten Ultrafiltration auf Membranen unterwirft, die die Phosphopeptide nicht zurückhalten, so daß diese in dieser Weise von Enzym getrennt werden und als Produkt gewonnen werden können.

2. Verfahren nach Anspruch 1, dadurch gekennzeichnet, daß man als Rohmaterial auf Basis von Casein Milch, einen Ultrafiltrationsrückstand der Milch oder auch vorher isolierte Phosphocaseinate von zweiwertigen Kationen, zum Beispiel Phosphocaseinate von Erdalkalimetallen, beispielsweise von Calcium oder Magnesium, verwendet.

3. Verfahren nach Anspruch 2, dadurch gekennzeichnet, daß man als Rohmaterial einen durch Ultrafiltration von Milch erhaltenes Retentat in einer Konzentrationshöhe von 1,5 bis 3, beispielsweise von etwa 2, verwendet, wobei diese Ultrafiltration von einer Diafiltration gefolgt sein kann.

4. Verfahren nach irgendeinem der Ansprüche 1 bis 3, dadurch gekennzeichnet, daß man die enzymatische Hydrolyse in einer Apparatur, die eine Ultrafiltrationsvorrichtung mit einem enzymatischen Reaktor kombiniert, durchführt und so einen enzymatischen Reaktor mit Membran darstellt.

5. Verfahren nach Anspruch 4, dadurch gekennzeichnet, daß man die enzymatische Hydrolyse kontinuierlich durchführt, indem man das Rohmaterial auf Basis von Casein in eine Reaktionszone mit dem Ziel eines innigen Inberührungsbringens mit dem Enzym einführt, das Reaktionsprodukt kontinuierlich abzieht, indem man es aus der Reaktionszone in eine Ultrafiltrationszone führt, aus der man ebenfalls kontinuierlich ein Permeat abzieht, das das Peptidhydrolysat darstellt.

6. Verfahren nach irgendeinem der Ansprüche 1 bis 5, dadurch gekennzeichnet, daß man den pH-Wert im Verlauf der enzymatischen Hydrolyse zwischen 7 und 9, vorzugsweise zwischen 7 und 8,5, beispielsweise auf etwa 8, einstellt.

7. Verfahren nach irgendeinem der Ansprüche 1 bis 6, dadurch gekennzeichnet, daß man als Enzym ein Pankreatin in Form eines natürlichen Pankreasextrakts oder synthetischen Gemischen von Trypsin und Alpha-Chymotrypsin verwendet.

8. Verfahren nach irgendeinem der Ansprüche 1 bis 7, dadurch gekennzeichnet, daß die Hydrolysentemperatur zwischen 37 und 45°C, vorzugsweise zwischen 37 und 40°C und vorteilhaft in der Größenordnung von 37°C gewählt wird.

9. Verfahren nach irgendeinem der Anspruch 1 bis 8, dadurch gekennzeichnet, daß man im enzymatischen Reaktor mit Membran Membranen verwendet, die das Enzym wirksam zurückzuhalten vermögen.

10. Verfahren nach irgendeinem der Ansprüche 1 bis 9, dadurch gekennzeichnet, daß nach der Hydrolyse die restliche Lösung des enzymatischen Reaktors einer Ultrafiltration auf Membranen, die die Phosphopeptide zurückzuhalten vermögen, während sie die nicht phosphorylierten Peptide durchlaufen lassen, beispielsweise auf Membranen, deren Schnittschwelle zwischen etwa 2000 und 50.000 liegt, unterworfen wird.

11. Verfahren nach Anspruch 10, dadurch gekennzeichnet, daß man auf die Ultrafiltration eine Diafiltration mit dem Ziel folgen läßt, die nicht phosphorylierte Peptidfraktion zu erschöpfen, indem man dem mit der Membran in Berührung gebrachten Produkt Wasser oder eine Salzlösung und vorteilhaft ein auf einen pH-Wert zwischen 7 und 8 gebrachtes Ultrafiltrat von Milch zusetzt.

12. Verfahren nach irgendeinem der Ansprüche 1 bis 11, dadurch gekennzeichnet, daß man die Zerfallsbehandlung der im Rückstand enthaltenen Phosphopeptide durch Ansäuern beispielsweise auf einem pH-Wert in der Nähe von 4,6 mit Hilfe einer anorganischen oder organischen Säure, die vorzugsweise auf dem Lebensmittelgebiet annehmbar ist, vorteilhaft Milchsäure, vornimmt.

13. Verfahren nach irgendeinem der Ansprüche 1 bis 11, dadurch gekennzeichnet, daß die

17

**0 033 686**

Zerfallsbehandlung der im Rückstand enthaltenen Phosphopeptide durch Einwirkung von Komplexbildnern des Calciums und/oder Magnesiums, beispielsweise Citraten, vorgenommen wird.

14. Verfahren nach irgendeinem der Ansprüche 1 bis 13, dadurch gekennzeichnet, daß man im Verlauf der letzten Stufe des Verfahrens physikalisch durch Ultrafiltration eine Fraktion abtrennt, die die gewünschten Phosphopeptide, Proteinrückstände und restliches Enzym enthält.

15. Verfahren nach irgendeinem der Ansprüche 1 bis 14, dadurch gekennzeichnet, daß man die Phosphopeptide in Salze von Phosphopeptiden und von Makroelementen, z.B. Calcium und/oder Magnesium, durch Inberührungsbringen der genannten Phosphopeptide mit Kationen oder Gemischen von Kationen der genannten Makroelemente überführt, was zu Organophosphorsalzen der genannten Makroelemente führt.

16. Verfahren nach Anspruch 15, dadurch gekennzeichnet, daß man die Makroelemente wenigstens teilweise durch Oligoelemente wie Eisen, Zunk, Kupfer, Chrom, Nickel, Kobalt, Mangan und Selen ersetzt, indem man bei der Reinigung der phosphopeptide eine Lösung verwendet, die das einzuführende oder die einzuführenden Oligoelemente enthält, was zu Organophosphorsalzen mit gebundenen Oligoelementen führt.

17. Organophosphorsalze von Phosphopeptiden und mineralischen Elementen, gekennzeichnet, durch die folgenden Parameter:

Summe der aromatischen Aminosäuren $<4\%$

$$\text{Verhältnis } \frac{Ca+Mg+P}{N_T} > 0,2$$

Gehalt an Serinen: über 8% und unter 20%
Gehalt an freien Aminosäuren: $<3\%$.

18. Nahrungsmittel oder Nahrungsmittelergänzungen, entsprechend den speziellen Ernährungsbedürfnissen und enthaltend ein Produkt gemäß Anspruch 17 oder ein Produkt, das sich aus dem Verfahren nach irgendeinem der Ansprüche 1 bis 16 ergibt.

19. Diätetische Lebensmittel oder Lebensmittel für die therapeutische Ernährung, enthaltend ein Produkt gemäß Anspruch 17 oder ein Produkt, das sich gemäß irgendeinem der Ansprüche 1 bis 16 ergibt, gegebenenfalls in Kombination mit einem für die orale oder enterale Ernährungs geeigneten neutralen Trägerstoff.

20. Nahrungsmittel nach Anspruch 19, enthaltend außerdem ergänzende Quellen von essentiellen Aminosäuren.

21. Arzneimittelzubereitungen, enthaltend ein Produkt gemäß Anspruch 17 oder ein Produkt, das sich aus dem Verfahren gemäß irgendeinem der Ansprüche 1 bis 16 ergibt.

22. Pharmazeutische Zusammensetzung, enthaltend eine wirksame Menge wenigstens eines Produkts gemäß Anspruch 17 oder eines Produkts, das sich aus dem Verfahren gemäß irgendeinem der Ansprüche 1 bis 16 ergibt, in Verbindung mit einem pharmazeutisch unbedenklichen Träger, wobei sich die Zubereitung insbesondere für die enterale Verabreichung eignet.

23. Zubereitung nach Anspruch 22, enthaltend wenigstens ein Phosphopeptidsalz gemäß Anspruch 17 oder ein Salz, das sich aus dem Verfahren nach Anspruch 15 ergibt, insbesondere von Calcium und/oder Magnesium, und eine anorganischem Phosphor, an Calcium und/oder Magnesium reiche Mineralstoff-Protein-Ergänzung darstellt.

24. Zubereitung nach Anspruch 22, enthaltend wenigstens ein aus dem Verfahren nach Anspruch 16 resultierendes Phosphopeptidsalz zum Heilen von digestiven intestinalen Resorptionsstörungen, die das Fehlen von Oligoelementen auslösen.

**Patentansprüche für den Vertragsstaat: AT**

1. Verfahren zur Behandlung eines Rohmaterials auf der Basis von Casein, das Phosphocaseinate von zweiwertigen Kationen, z.B. Phosphocaseinate von Calcium und/oder Magnesium, enthält, dadurch gekennzeichnet, daß man das Rohmaterial einer enzymatischen Hydrolyse mit Hilfe wenigstens eines proteolytischen Enzyms unterwirft, das die in vivo im menschlichen Organismus stattfindende Proteinverdauung wieder zu erzeugen vermag, daß man das erhaltene Hydrolysat gewinnt, indem man dieses wenigstens einer Ultrafiltration auf Membranen unterwirft, die die Phosphopeptide zurückzuhalten vermögen, während sie die Peptide durchgehen lassen, so daß das Ultrafiltrat in dieser Weise die nicht phosphorylierten Peptide enthält, daß man den Ultrafiltrationsrückstand gewinnt, daß man eine Zerfallsbehandlung der im Rückstand enthaltenen Phosphopeptide vornimmt und daß man den Rückstand wenigstens einer erneuten Ultrafiltration auf Membranen unterwirft, die die Phosphopeptide nicht zurückhalten, so daß diese in dieser Weise vom Enzym getrennt werden und als Produkt gewonnen werden können.

2. Verfahren nach Anspruch 1, dadurch gekennzeichnet, daß man als Rohmaterial auf Basis von Casein Milch, einen Ultrafiltrationsrückstand der Milch oder auch vorher isolierte Phosphocaseinate von zweiwertigen Kationen, zum Beispiel Phosphocaseinate von Erdalkalimetallen, beispielsweise von Calcium oder Magnesium, verwendet.

3. Verfahren nach Anspruch 2, dadurch gekennzeichnet, daß man als Rohmaterial einen durch Ultrafiltration von Milch erhaltenes Retentat in einer Konzentrationshöhe von 1,5 bis 3, beispielsweise von etwa 2, verwendet, wobei diese Ultrafiltration von einer Diafiltration gefolgt sein kann.

4. Verfahren nach irgendeinem der Ansprüche 1 bis 3, dadurch gekennzeichnet, daß man die enzymatische Hydrolyse in einer Apparatur, die ein Ultrafiltrationsvorrichtung mit einem enzymatischen Reaktor kombiniert, durchführt und so einen enzymatischen Reaktor mit Membran darstellt.

5. Verfahren nach Anspruch 4, dadurch gekennzeichnet, daß man die enzymatische Hydrolyse kontinuierlich durchführt, indem man das Rohmaterial auf Basis von Casein in eine Reaktionszone mit dem Ziel eines innigen Inberührungbringens mit dem Enzym einführt, das Reaktionsprodukt kontinuierlich abzieht, indem man es aus der Reaktionszone in eine Ultrafiltrationszone führt, aus der man ebenfalls kontinuierlich ein Permeat abzieht, das das Peptidhydrolysat darstellt.

6. Verfahren nach irgendeinem der Ansprüche 1 bis 5, dadurch gekennzeichnet, daß man den pH-Wert im Verlauf der enzymatischen Hydrolyse zwischen 7 und 9, vorzugsweise zwischen 7 und 8,5, beispielsweise auf etwa 8, einstellt.

7. Verfahren nach irgendeinem der Ansprüche 1 bis 6, dadurch gekennzeichnet, daß man als Enzym ein Pankreatin in Form eines natürlichen Pankreasextrakts oder synthetischen Gemischen von Trypsin und Alpha-Chymotrypsin verwendet.

8. Verfahren nach irgendeinem der Ansprüche 1 bis 7, dadurch gekennzeichnet, daß die Hydrolysentemperatur zwischen 37 und 45°C, vorzugsweise zwischen 37 und 40°C und vorteilhaft in der Größenordnung von 37°C gewählt wird.

9. Verfahren nach irgendeinem der Anspruch 1 bis 8, dadurch gekennzeichnet, daß man im enzymatischen Reaktor mit Membran Membranen verwendet, die das Enzym wirksam zurückzuhalten vermögen.

10. Verfahren nach irgendeinem der Ansprüche 1 bis 9, dadurch gekennzeichnet, daß nach der Hydrolyse die restliche Lösung des enzymatischen Reaktors einer Ultrafiltration auf Membranen, die die Phosphopeptide zurückzuhalten vermögen, während sie die nicht phosphorylierten Peptide durchlaufen lassen, beispielsweise auf Membranen, deren Schnittschwelle zwischen etwa 2000 und 50.000 liegt, unterworfen wird.

11. Verfahren nach Anspruch 10, dadurch gekennzeichnet, daß man auf die Ultrafiltration eine Diafiltration mit dem Ziel folgen läßt, die nicht phosphorylierte Peptidfraktion zu erschöpfen, indem man dem mit der Membran in Berührung gebrachten Produkt Wasser oder eine Salzlösung und vorteilhaft ein auf einen pH-Wert zwischen 7 und 8 gebrachtes Ultrafiltrat von Milch zusetzt.

12. Verfahren nach irgendeinem der Ansprüche 1 bis 11, dadurch gekennzeichnet, daß man die Zerfallsbehandlung der im Rückstand enthaltenen Phosphopeptide durch Ansäuern beispielsweise auf einem pH-Wert in der Nähe von 4,6 mit Hilfe einer anorganischen oder organischen Säure, die vorzugsweise auf dem Lebensmittelgebiet annehmbar ist, vorteilhaft Milchsäure, vornimmt.

13. Verfahren nach irgendeinem der Ansprüche 1 bis 11, dadurch gekennzeichnet, daß die Zerfallsbehandlung der im Rückstand enthaltenen Phosphopeptide durch Einwirkung von Komplexbildnern des Calciums und/oder Magnesiums, beispielsweise Citraten, vorgenommen wird.

14. Verfahren nach irgendeinem der Ansprüche 1 bis 13, dadurch gekennzeichnet, daß man im Verlauf der letzten Stufe des Verfahrens physikalisch durch Ultrafiltration eine Fraktion abtrennt, die die gewünschten Phosphopeptide, Proteinrückstände und restliches Enzym enthält.

15. Verfahren nach irgendeinem der Ansprüche 1 bis 14, dadurch gekennzeichnet, daß man die Phosphopeptide in Salze von Phosphopeptiden und von Makroelementen, z.B. Calcium und/oder Magnesium, durch Inberührungbringen der genannten Phosphopeptide mit Kationen oder Gemischen von Kationen der genannten Makroelemente überführt, was zu Organophosphorsalzen der genannten Makroelemente führt.

16. Verfahren nach Anspruch 15, dadurch gekennzeichnet, dass man die Makroelemente wenigstens teilweise durch Oligoelemente wie Eisen, Zink, Kupfer, Chrom, Nickel, Kobalt, Mangan und Selen ersetzt, indem man bei der Reinigung der Phosphopeptide eine Lösung verwendet, die das einzuführende oder die einzuführenden Oligoelemente enthält, was zu Organophosphorsalzen mit gebundenen Oligoelementen fürhrt.

17. Verfahren nach irgendeinem der vorherigen Ansprüche dadurch gekennzeichnet, dass man Organophosphorsalze von Phosphopeptiden und mineralischen Elementen, gekennzeichnet durch die folgenden Parameter:

Summe der aromatischen Aminosäuren <4%

$$\text{Verhältnis} \quad \frac{Ca+Mg+P}{N_T} \quad >0,2$$

Gehalt an Serinen: über 8% und unter 20%
Gehalt an freien Aminosäuren: <3%, herstellt.

**O033686**

18. Verfahren zur Herstellung von Nahrungsmittel oder Nahrungsmittelergänzungen, entsprechend den speziellen Ernährungsbedürfnissen, dadurch gekennzeichnet, dass man ein Produkt zugibt das sich aus dem Verfahren nach irgendeinem der Ansprüche 1 bis 17 ergibt.

19. Verfahren zur Herstellung von diätetische Lebensmittel oder Lebensmittel für die therapeutische Ernährung, enthaltend ein Produkt, das sich gemäss irgendeinem der Ansprüche 1 bis 18 ergibt, gegebenenfalls in Kombination mit einem für die orale oder enterale Ernährung geeigneten neutralen Trägerstoff.

20. Verfahren zur Herstellung von nach Anspruch 9 hergestellten Nahrungsmittel, enthaltend ausserdem ergänzende Quellen von essentiellen Aminosäuren.

21. Verfahren zur Herstellung von einer pharmazeutischen Zusammensetzung, enthaltend eine wirksame Menge wenigstens eines produckts das sich aus dem Verfahren gemäss irgendeinem der Ansprüche 1 bis 17 ergibt, dadurch gekennzeichnet, dass man dieses Produkt mit einem pharmazeutisch unbedenklichen Träger Kombiniert, wobei sich die Zubereitung insbesondere fürenterale Verabreichung eignet.

22. Verfahren zur Herstellung einer Zubereitung nach Anspruch 21, enthaltend wenigstens ein Phosphopeptidsalz, insbesondere von Calcium und/oder Magnesium, das eine an organischem Phosphor, an Calcium und/oder Magnesium reiche Mineralstoff-Protein-Ergänzung darstellt, dadurch gekennzeichnet, dass dieses Salz nach Anspruch 15 oder 17 hergestellt ist.

**Claims for the contracting States: BE, CH, DE, FR, GB, IT, LI, LU, NL, SE**

1. Process for treating a raw material based on casein containing phosphocaseinates of divalent cations, such as calcium and/or magnesium phosphocaseinates, characterized in that the raw material is subjected to an enzymatic hydrolysis by means of at least one proteolytic enzyme able of reproducing the proteic digestion which occurs in vivo in the human body, that the resulting hydrolyzate is recovered, that said hydrolyzate is subjected to at least one ultrafiltration on membranes capable of retaining the phosphopeptides while letting the peptides pass therethrough, the ultrafiltrate thus containing the non phosphorylated peptides, that the ultrafiltration retentate is recovered, that a treatment is effected to disaggregate the phosphopeptides contained in said retentate, and that the latter is subjected to at least one further ultrafiltration on membranes which do not retain the phosphopeptides, these being thus separated from the enzyme and available to be recovered as a product.

2. Process according to claim 1, characterized in that, as a raw material based on casein, there is used milk, or a milk ultrafiltration retentate, or phosphocaseinates of divalent cations previously isolated, such as phosphocaseinates of alkaline-earth metals, such as calcium or magnesium.

3. Process according to claim 2, characterized in that there is used as a raw material a retentate obtained by milk ultrafiltration, at a concentration ratio ranging from 1.5 to 3, for instance of about 2, said ultrafiltration being possibly followed by a diafilatration.

4. Process according to any of claims 1 to 3, characterized in that the enzymatic hydrolysis is made in a device which combines an ultrafiltration equipment with an enzymatic reactor, thus forming a membrane type enzymatic reactor.

5. Process according to claim 4, characterized in that the enzymatic hydrolysis is effected continuously by feeding the raw material based on casein to a reaction zone to bring it into intimate contact with the enzyme, the reaction product is withdrawn continuously and transferred from the reaction zone to an ultrafiltration zone, wherefrom there is also withdrawn continuously a permeate which represents the peptidic hydrolysate.

6. Process according to any of claims 1 to 5, characterized in that the pH is adjusted in the range of 7 to 9 and preferably of 7 to 8.5, for example to about 8, during said enzymatic hydrolysis.

7. Process according to any of claims 1 to 6, characterized in that there is used as an enzyme a pancreatin under the form of a natural pancreatic extract, or synthetic mixtures of trypsine and alphachymotrypsine.

8. Process according to any of claims 1 to 7, characterized in that the hydrolysis temperature is selected in the range of 37 to 45°C and preferably of 37 to 40°C and is, to advantage, of the order of 37°C.

9. Process according to any of claims 1 to 8, characterized in that, in the membrane-type enzymatic reactor, use is made of membranes capable of retaining efficiously the enzyme.

10. Process according to any of claims 1 to 9, characterized in that, after said hydrolysis, the residual solution of the enzymatic reactor is subjected to ultrafiltration on membranes capable of retaining the phosphopeptides, while letting the non phosphorylated peptides pass therethrough, such as membranes having a cut-off threshold in the range of about 2000 to 50.000.

11. Process according to claim 10, characterized in that said ultrafiltration is followed by a dialfiltration for depleting the non phosphorylated peptidic fraction by adding to the product brought into contact with the membrane either water or salt-containing water solution or advantageously, a milk ultrafiltrate with a pH raised to between 7 and 8.

12. Process according to any of claims 1 to 11, characterized in that the treatment for disaggregating the phosphopeptides contained in the retentate is effected by acidification; as example to a pH of about 4.6, by means of an inorganic or organic acid, preferably an acid accepted in the nutritional field, advantageously lactic acid.

13. Process according to any of claims 1 to 11, characterized in that the treatment for disaggregating the phosphopeptides in the retentate is effected by action of complexing agents for calcium and/or magnesium, such as citrates.

14. Process according to any of claims 1 to 13, characterized in that, during the last stage of the process, there is effected, by ultrafiltration, physical separation of a fraction containing the desired phosphopeptides from the proteic residues and the residual enzyme.

15. Process according to any of claims 1 to 14, characterized in that phosphopeptides are converted into salts of said phosphopeptides and macroelements, such as calcium and/or magnesium, by putting into presence said phosphopeptides with cations or mixtures of cations of said macroelements, whereby are obtained organophosphorated salts of said macroelements.

16. Process according to claim 15, characterized in that at least partially the macroelements are replaced by oligoelements, such as iron, zinc, copper, chromium, nickel, cobalt, manganese and selenium, by using, when purifying the phosphopeptides, a solution containing one or more oligoelements to be introduced, whereby organophosphorated salts with linked oligoelements are obtained.

17. Organophosphorated salts of phosphopeptides and mineral elements characterized by the following parameters:

— total of aromatic amino-acids <4%

$$— \text{ratio } \frac{Ca+Mg+P}{N_T} > 0.2$$

— serines ratio higher than 8% and lower than 20%
— free amino-acids content <3%.

18. Food or food additives useful for specific nutritional requirements and containing a product according to claim 17 or a product obtained by the process according to any of claims 1 to 16.

19. Food for dietetic or for therapeutic nutrition containing a product according to claim 17 or a product obtained by the process according to any of claims 1 to 16 possibly in association with a neutral carrier suitable for oral or enteral alimentation.

20. Foods according to claim 19 further containing complementary sources of essential aminoacids.

21. Drugs containing a product according to claim 17 or a product obtained by the process according to any of claims 1 to 16.

22. Pharmaceutical composition containing an efficient quantity of at least one product according to claim 17 on a product obtained by the process according to any of claims 1 to 16 in association with a pharmaceutically acceptable carrier, suitable more particularly to enteral administration.

23. Composition according to claim 22, containing at least a phosphopeptide salt according to claim 17 or obtained by the process according to claim 15, more particularly of calcium and/or magnesium, constituting a mineralo-protidic supplement rich in organic phosphorus, in calcium and/or magnesium.

24. Composition according to claim 22 containing at least a phosphopeptide salt resulting from the process of claim 16, to cure digestive bad absorptions inducing lacks of oligoelements.

## Claims for the contracting State: AT

1. Process for treating a raw material based on casein containing phosphocaseinates of divalent cations, such as calcium and/or magnesium phosphocaseinates, characterized in that the raw material is subjected to an enzymatic hydrolysis by means of at least one proteolytic enzyme able of reproducing the proteic digestion which occurs in vivo in the human body, that the resulting hydrolyzate is recovered, that said hydrolyzate is subjected to at least one ultrafiltration on membranes capable of retaining the phosphopeptides while letting the peptides pass therethrough, the ultrafiltrate thus containing the non phosphorylated peptides, that the ultrafiltration retentate is recovered, that a treatment is effected to disaggregate the phosphopeptides contained in said retentate, and that the latter is subjected to at least one further ultrafiltration on membranes which do not retain the phosphopeptides, these being thus separated from the enzyme and available to be recovered as a product.

2. Process according to claim 1, characterized in that, as a raw material based on casein, there is used milk, or a milk ultrafiltration retentate, or phosphocaseinates of divalent cations previously isolated, such as phosphocaseinates of alkaline-earth metals, such as calcium or magnesium.

21

**0 033 686**

3. Process according to claim 2, characterized in that there is used as a raw material a retentate obtained by milk ultrafiltration, at a concentration ratio ranging from 1.5 to 3, for instance of about 2, said ultrafiltration being possibly followed by a diafiltration.

4. Process according to any of claims 1 to 3, characterized in that the enzymatic hydrolysis is made in a device which combines an ultrafiltration equipment with an enzymatic reactor, thus forming a membrane type enzymatic reactor.

5. Process according to claim 4, characterized in that the enzymatic hydrolysis is effected continuously by feeding the raw material based on casein to a reaction zone to bring it into intimate contact with the enzyme, the reaction product is withdrawn continuously and transferred from the reaction zone to an ultrafiltration zone, wherefrom there is also withdrawn continuously a permeate which represents the peptidic hydrolysate.

6. Process according to any of claims 1 to 5, characterized in that the pH is adjusted in the range of 7 to 9 and preferably of 7 to 8.5, for example to about 8, during said enzymatic hydrolysis.

7. Process according to any of claims 1 to 6, characterized in that there is used as an enzyme a pancreatin under the form of a natural pancreatic extract, or synthetic mixtures of trypsine and alphachymotrypsine.

8. Process according to any of claims 1 to 7, characterized in that the hydrolysis temperature is selected in the range of 37 to 45°C and preferably of 37 to 40°C and is, to advantage, of the order of 37°C.

9. Process according to any of claims 1 to 8, characterized in that, in the membrane-type enzymatic reactor, use is made of membranes capable of retaining efficiously the enzyme.

10. Process according to any of claims 1 to 9, characterized in that, after said hydrolysis, the residual solution of the enzymatic reactor is subjected to ultrafiltration on membranes capable of retaining the phosphopeptides, while letting the non phosphorylated peptides pass therethrough, such as membranes having a cut-off threshold in the range of about 2000 to 50.000.

11. Process according to claim 10, characterized in that said ultrafiltration is followed by a dialfiltration for depleting the non phosphorylated peptidic fraction by adding to the product brought into contact with the membrane either water or salt-containing water solution or advantageously, a milk ultrafiltrate with a pH raised to between 7 and 8.

12. Process according to any of claims 1 to 11, characterized in that the treatment for disaggregating the phosphopeptides contained in the retentate is effected by acidification, as example to a pH of about 4.6, by means of an inorganic or organic acid, preferably an acid accepted in the nutritional field, advantageously lactic acid.

13. Process according to any of claims 1 to 11, characterized in that the treatment for disaggregating the phosphopeptides in the retentate is effected by action of complexing agents for calcium and/or magnesium, such as citrates.

14. Process according to any of claims 1 to 13, characterized in that, during the last stage of the process, there is effected, by ultrafiltration, physical separation of a fraction containing the desired phosphopeptides from the proteic residues and the residual enzyme.

15. Process according to any of claims 1 to 14, characterized in that phosphopeptides are converted into salts of said phosphopeptides and macroelements, such as calcium and/or magnesium, by putting into presence said phosphopeptides with cations or mixtures of cations of said macroelements, whereby are obtained organophosphorated salts of said macroelements.

16. Process according to claim 15, characterized in that at least partially the macroelements are replaced by oligoelements, such as iron, zinc, copper, chromium, nickel, cobalt, manganese and selenium, by using, when purifying the phosphopeptides, a solution containing one or more oligoelements to be introduced, whereby organophosphorated salts with linked oligoelements are obtained.

17. Process according to any of preceding claims, characterized in that organophosphorated salts of phosphopeptides and minerals elements characterized by the following parameters:

— total of aromatic amino-acids <4%

$$— \text{ratio} \frac{Ca+Mg+P}{N_T} > 0.2$$

— serines ratio higher than 8% and lower than 20%
— free amino-acids content <3% are prepared.

18. Process for preparing food or food additives useful for specific nutritional requirements characterized in that a product obtained by the process according to any of claims 1 to 17 is introduced therein.

19. Process for preparing food for dietetic or for therapeutic nutrition containing a product obtained by the process according to any of claims 1 to 18 possibly in association with a neutral carrier suitable for oral or enteral alimentation.

22

20. Process for preparing foods obtained according to claim 19 further containing complementary sources of essential aminoacids.

21. Process for preparing a pharmaceutical composition containing an efficient quantity of at least one product obtained by the process according to any of claims 1 to 17 characterized in that said product is associated with a pharmaceutically acceptable carrier, suitable more particularly to enteral administration.

22. Process for preparing a composition according to claim 21, containing at least a phosphopeptide salt more particularly of calcium and/or magnesium, constituting a mineralo-protidic supplement rich in organic phosphorus, in calcium and/or magnesium, characterized in that said salt is prepared according to claims 15 or 17.

FIG.1

FIG.2

FIG. 3